# EUROPEAN PATENT APPLICATION

(11) **EP 4 821 511 A1**
(43) Date of publication of application: **14.10.2026**
(21) Application number: 25382367.8
(22) Date of filing: 09.04.2025
(51) Int. Cl.: A61K 48/00, C12N 9/10, C12N 15/86

(54) **NUCLEIC ACID VECTOR COMPRISING A CODING SEQUENCE OF THE EPM2B GENE**

(71) Applicant: Fundacion Instituto De Investigacion Sanitaria Fundacion Jimenez Diaz, 28040 Madrid (ES)
(72) Inventor: SERRATOSA FERNÁNDEZ, José María, 28040 Madrid (ES); ZAFRA PUERTA, Luis, 28040 Madrid (ES); SÁNCHEZ GARCÍA, Marina, 28040 Madrid (ES)
(74) Representative: Hoffmann Eitle

(57) **Abstract**

The present invention refers to a nucleic acid vector to be used in the context of gene therapy in the treatment of Lafora disease.

## Description

### FIELD OF THE INVENTION

The present invention refers to the medical field. Particularly, it refers to a nucleic acid vector to be used in the context of gene therapy, particulary in the treatment of Lafora disease (LD).

### STATE OF THE ART

LD (OMIM#254780; ORPHA:501) is a rare, progressive neurodegenerative disorder that initially presents with cognitive deficits, myoclonus, and visual and generalized tonic-clonic seizures. The disease typically manifests in late childhood or early adolescence, followed by a rapid decline characterized by severe dementia, ataxia, dysarthria, aphasia, visual impairment, and respiratory failure. This progressive deterioration ultimately results in a vegetative state and death, usually within a decade of symptom onset.

LD belongs to the group of glycogen storage diseases and is associated with the accumulation of Lafora bodies (LBs), which are composed of polyglucosans (abnormal, insoluble, and poorly branched forms of glycogen) along with various proteins. The presence of LBs is a hallmark of the disease and is believed to contribute to its pathophysiology.

Multiple preclinical models have been developed to study LD and evaluate potential therapeutic strategies. Various pharmacological and genetic approaches, including AMPK activators, antisense oligonucleotides, neuroinflammation modulators, and gene therapy strategies, have been explored in these models. Despite promising advances, there remains an urgent need for effective and reliable therapeutic options for LD.

The present invention addresses this critical need by providing a novel therapeutic strategy aimed at mitigating the progression of LD.

### DESCRIPTION OF THE INVENTION

### Brief description of the invention

The present invention refers to a nucleic acid vector to be used in the context of gene therapy, particularly in the treatment of LD.

According to the results provided in **Example 2,** the present invention demonstrates the efficacy of rAAV-hEPM2B (ICV-administered vector at 3 months of age) gene therapy in addressing molecular and pathological hallmarks associated with Epm2b deficiency. The intracerebroventricular (ICV) administration of rAAV-hEPM2B or rAAV-GFP vectors resulted in efficient transgene transcription and translation within the brain, highlighting the robust potential of this delivery approach for targeted gene expression in the central nervous system (CNS).

Following intravenous (IV) administration of the rAAVP31-hEPM2B vector (IV-administered vector at 1 month of age) in Epm2b-/- mice, widespread transduction of CNS cells and peripheral organs was observed, indicating successful systemic delivery and transgene expression. The therapeutic impact of rAAVP31-hEPM2B was further evidenced by its ability to reduce astrogliosis, mitigate neurodegeneration, and decrease the formation of Lafora bodies (LBs) in treated mice. These findings suggest that systemic delivery of rAAVP31-hEPM2B contributes to neuropathological improvements, potentially altering disease progression.

In addition to its effects on cellular pathology, rAAV-hEPM2B gene therapy demonstrated a capacity to modulate neuroinflammatory responses. ICV administration of rAAV-hEPM2B led to a significant reduction in astrogliosis and microgliosis, while IV administration decreased neuroinflammation marker levels in Epm2b-/- mice. The attenuation of neuroinflammatory responses suggests that rAAV-hEPM2B may exert a protective role against inflammatory-mediated neuronal damage.

Behavioral analyses revealed that rAAV-hEPM2B treatment effectively reversed episodic memory deficits and enhanced spontaneous locomotor activity in Epm2b-/- mice. However, motor coordination impairments remained uncorrected, indicating that while the therapy addresses some functional deficits, further optimization may be required to fully restore motor performance. These findings underscore the differential impact of rAAV-hEPM2B therapy across various neurological domains.

Electrophysiological assessments in the dentate gyrus (DG) and striatum of both wild-type (WT) and Epm2b-/- mice following IV administration of rAAVP31-hEPM2B provided further insights into the neurophysiological effects of the therapy. While additional studies are needed to fully characterize these changes, preliminary data suggest potential normalization of synaptic function.

Finally, the systemic administration of rAAVP31-hEPM2B prompted an evaluation of its effects on liver function and immune responses. These findings highlight the therapeutic effect of rAAV-hEPM2B gene therapy while emphasizing the need for continued investigation to optimize efficacy and safety profiles for potential clinical translation.

Particulalry, the inventors of the present invention have demsotrated that aberrant aggregates in 6-month-old *Epm2b*^{*-*/*-*} mice treated IV with rAAVP31-h*EPM2B* at early stages, are significantly reduced in the number of LBs in the CA1 and CA2-CA3 regions of the hippocampus, 5 months after IV administration. These findings indicate that early systemic administration of rAAVP31-h*EPM2B* is more effective in regulating glycogen metabolism alterations compared to ICV administration of the rAAV-h*EPM2B* vectors at three months of age. This was due to the inability to form the laforin-malin complex, which is essential for interacting with enzymes that regulate glycogen metabolism. This failure likely resulted from laforin being trapped within the LBs in 3-month-old *Epm2b* mice, rendering malin administration insufficient to halt the formation of LBs. In contrast, *Epm2b*^{*-*/*-*} mice at one month of age exhibit very few LBs, which may allow for a greater proportion of free, active laforin compared to older mice. The effectiveness of the therapy in preventing LB formation at one month of age supports this hypothesis.

So, the first embodiment of the present invention refers to a vector (hereinafter "vector of the invention", for instance rAAV-hEPM2B or rAAVP31-hEPM2B) comprising a nucleic acid sequence coding for EPM2B protein (malin protein).

In a preferred embodiment, the vector of the invention is characterized in that it is a nucleic acid-based vector, nanoparticle-based vector, extracellular vesicles or cell-derived vector.

In a preferred embodiment, the nucleic acid-vector is a non-viral nucleic acid vector or a viral nucleic acid vector.

In a preferred embodiment, the nucleic acid-vector is a non-viral nucleic acid vector selected from: plasmids, BACs (Bacterial Artificial Chromosomes), YACs (Yeast Artificial Chromosomes), mRNA (messenger RNA) or saRNA (self-amplifying RNA), or a viral nucleic acid vector selected from: adenovirus, lentivirus, adenovirus, retrovirus or HSV ((Herpes Simplex Virus).

In a preferred embodiment, the EPM2B protein is encoded by a nucleic acid sequence comprising the sequence SEQ ID NO: 1:

ATGGCGGCCGAAGCCTCGGAGAGCGGGCCAGCGCTGCATGAGCTCATGCGCGAG
GCGGAGATCAGCCTGCTCGAGTGCAAGGTGTGCTTTGAGAAGTTTGGCCACCGG
CAGCAGCGGCGCCCGCGCAACCTGTCCTGCGGCCACGTGGTCTGCCTGGCCTGCG
TGGCCGCCCTGGCGCACCCGCGCACTCTGGCCCTCGAGTGCCCATTCTGCAGGCG
AGCTTGCCGGGGCTGCGACACCAGCGACTGCCTGCCGGTGCTGCACCTCATAGA
GCTCCTGGGCTCAGCGCTTCGCCAGTCCCCGGCCGCCCATCGCGCCGCCCCCAGC
GCCCCCGGAGCCCTCACCTGCCACCACACCTTCGGCGGCTGGGGGACCCTGGTCA
ACCCCACCGGACTGGCGCTTTGTCCCAAGACGGGGCGTGTCGTGGTGGTGCACG
ACGGCAGGAGGCGTGTCAAGATTTTTGACTCAGGGGGAGGATGCGCGCATCAGT
TTGGAGAGAAGGGGGACGCTGCCCAAGACATTAGGTACCCTGTGGATGTCACCA
TCACCAACGACTGCCATGTGGTTGTCACTGACGCCGGCGATCGCTCCATCAAAGT
GTTTGATTTTTTTGGCCAGATCAAGCTTGTCATTGGAGGCCAATTCTCCTTACCTT
GGGGTGTGGAGACCACCCCTCAGAATGGGATTGTGGTAACTGATGCGGAGGCAG
GGTCCCTGCACCTCCTGGACGTCGACTTCGCGGAAGGGGTCCTTCGGAGAACTGA
AAGGTTGCAAGCTCATCTGTGCAATCCCCGAGGGGTGGCAGTGTCTTGGCTCACC
GGGGCCATTGCGGTCCTGGAGCACCCCCTGGCCCTGGGGACTGGGGTTTGCAGC
ACCAGGGTGAAAGTGTTTAGCTCAAGTATGCAGCTTGTCGGCCAAGTGGATACCT
TTGGGCTGAGCCTCTACTTTCCCTCCAAAATAACTGCCTCCGCTGTGACCTTTGAT
CACCAGGGAAATGTGATTGTTGCAGATACATCTGGTCCAGCTATCCTTTGCTTAG
GAAAACCTGAGGAGTTTCCAGTACCGAAGCCCATGGTCACTCATGGTCTTTCGCA
TCCTGTGGCTCTTACCTTCACCAAGGAGAATTCTCTTCTTGTGCTGGACACAGCAT
CTCATTCTATAAAAGTCTATAAAGTTGACTGGGGGTGA

In a preferred embodiment, the EPM2B protein comprises the amino acid sequence SEQ ID NO: 2:

In a preferred embodiment, the vector of the invention comprises the SEQ ID NO: 3 (plasmid sequence), wherein CAG promoter is underlined and the transgen hEPM2B in bold and undelined **(bold)**.

CAGCTGCGCGCTCGCTCGCTCACTGAGGCCGCCCGGGCAAAGCCCGGGCGTCGG
GCGACCTTTGGTCGCCCGGCCTCAGTGAGCGAGCGAGCGCGCAGAGAGGGAGTG
GCCAACTCCATCACTAGGGGTTCCTTGTAGTTAATGATTAACCCGCCATGCTACT
TATCTACTCGACATTGATTATTGACTAGTTATTAATAGTAATCAATTACGGGGTC
ATTAGTTCATAGCCCATATATGGAGTTCCGCGTTACATAACTTACGGTAAATGGC
CCGCCTGGCTGACCGCCCAACGACCCCCGCCCATTGACGTCAATAATGACGTATG
TTCCCATAGTAACGCCAATAGGGACTTTCCATTGACGTCAATGGGTGGAGTATTT
ACGGTAAACTGCCCACTTGGCAGTACATCAAGTGTATCATATGCCAAGTACGCCC
CCTATTGACGTCAATGACGGTAAATGGCCCGCCTGGCATTATGCCCAGTACATGA
CCTTATGGGACTTTCCTACTTGGCAGTACATCTACGTATTAGTCATCGCTATTACC
ATGGTCGAGGTGAGCCCCACGTTCTGCTTCACTCTCCCCATCTCCCCCCCCTCCCC
ACCCCCAATTTTGTATTTATTTATTTTTTAATTATTTTGTGCAGCGATGGGGGCGG
GGGGGGGGGGGGCGCGCGCCAGGCGGGGCGGGGCGGGGCGAGGGGCGGGG
CGGGGCGAGGCGGAGAGGTGCGGCGGCAGCCAATCAGAGCGGCGCGCTCCGAA
AGTTTCCTTTTATGGCGAGGCGGCGGCGGCGGCGGCCCTATAAAAAGCGAAGCG
CGCGGCGGGCGGGAGTCGCTGCGTTGCCTTCGCCCCGTGCCCCGCTCCGCGCCGC
CTCGCGCCGCCCGCCCCGGCTCTGACTGACCGCGTTACTCCCACAGGTGAGCGGG
CGGGACGGCCCTTCTCCTCCGGGCTGTAATTAGCGCTTGGTTTAATGACGGCTTG
TTTCTTTTCTGTGGCTGCGTGAAAGCCTTGAGGGGCTCCGGGAGGGCCCTTTGTG
CGGGGGGAGCGGCTCGGGGGGTGCGTGCGTGTGTGTGTGCGTGGGGAGCGCCGC
GTGCGGCTCCGCGCTGCCCGGCGGCTGTGAGCGCTGCGGGCGCGGCGCGGGGCT
TTGTGCGCTCCGCAGTGTGCGCGAGGGGAGCGCGGCCGGGGGCGGTGCCCCGCG
GTGCGGGGGGGCTGCGAGGGGAACAAAGGCTGCGTGCGGGGTGTGTGCGTGGGG
GGGTGAGCAGGGGGTGTGGGCGCGTCGGTCGGGCTGCAACCCCCCCTGCACCCC
CCTCCCCGAGTTGCTGAGCACGGCCCGGCTTCGGGTGCGGGGCTCCGTACGGGG
CGTGGCGCGGGGCTCGCCGTGCCGGGCGGGGGGTGGCGGCAGGTGGGGGTGCCG
GGCGGGGCGGGGCCGCCTCGGGCCGGGGAGGGCTCGGGGGAGGGGCGCGGCGG
CCCCCGGAGCGCCGGCGGCTGTCGAGGCGCGGCGAGCCGCAGCCATTGCCTTTT

ATGGTAATCGTGCGAGAGGGCGCAGGGACTTCCTTTGTCCCAAATCTGTGCGGA
GCCGAAATCTGGGAGGCGCCGCCGCACCCCCTCTAGCGGGCGCGGGGCGAAGCG
GTGCGGCGCCGGCAGGAAGGAAATGGGCGGGGAGGGCCTTCGTGCGTCGCCGCG
CCGCCGTCCCCTTCTCCCTCTCCAGCCTCGGGGCTGTCCGCGGGGGGACGGCTGC
CTTCGGGGGGGACGGGGCAGGGCGGGGTTCGGCTTCTGGCGTGTGACCGGCGGC
TCTAGAGCCTCTGCTAACCATGTTCATGCCTTCTTCTTTTTCCTACAGCTCCTGGG
CAACGTGCTGGTTATTGTGCTGTCTCATCATTTTGGCAAAGAATTGATTAATTCGA
GCGAACGCGTCGAGTCGCTCGGTACGATTTAAATTGaattggcctcgagcgcaagcttgagctagc
ATGGCGGCCGAAGCCTCGGAGAGCGGGCCAGCGCTGCATGAGCTCATGCG
CGAGGCGGAGATCAGCCTGCTCGAGTGCAAGGTGTGCTTTGAGAAGTTTGG
CCACCGGCAGCAGCGGCGCCCGCGCAACCTGTCCTGCGGCCACGTGGTCTG
CCTGGCCTGCGTGGCCGCCCTGGCGCACCCGCGCACTCTGGCCCTCGAGTG
CCCATTCTGCAGGCGAGCTTGCCGGGGCTGCGACACCAGCGACTGCCTGCC
GGTGCTGCACCTCATAGAGCTCCTGGGCTCAGCGCTTCGCCAGTCCCCGGC
CGCCCATCGCGCCGCCCCCAGCGCCCCCGGAGCCCTCACCTGCCACCACAC
CTTCGGCGGCTGGGGGACCCTGGTCAACCCCACCGGACTGGCGCTTTGTCC
CAAGACGGGGCGTGTCGTGGTGGTGCACGACGGCAGGAGGCGTGTCAAGA
TTTTTGACTCAGGGGGAGGATGCGCGCATCAGTTTGGAGAGAAGGGGGACG
CTGCCCAAGACATTAGGTACCCTGTGGATGTCACCATCACCAACGACTGCCA
TGTGGTTGTCACTGACGCCGGCGATCGCTCCATCAAAGTGTTTGATTTTTT
GGCCAGATCAAGCTTGTCATTGGAGGCCAATTCTCCTTACCTTGGGGTGTG
GAGACCACCCCTCAGAATGGGATTGTGGTAACTGATGCGGAGGCAGGGTCC
CTGCACCTCCTGGACGTCGACTTCGCGGAAGGGGTCCTTCGGAGAACTGAA
AGGTTGCAAGCTCATCTGTGCAATCCCCGAGGGGTGGCAGTGTCTTGGCTC
ACCGGGGCCATTGCGGTCCTGGAGCACCCCCTGGCCCTGGGGACTGGGGTT
TGCAGCACCAGGGTGAAAGTGTTTAGCTCAAGTATGCAGCTTGTCGGCCAA
GTGGATACCTTTGGGCTGAGCCTCTACTTTCCCTCCAAAATAACTGCCTCCG
CTGTGACCTTTGATCACCAGGGAAATGTGATTGTTGCAGATACATCTGGTCC
AGCTATCCTTTGCTTAGGAAAACCTGAGGAGTTTCCAGTACCGAAGCCCATG
GTCACTCATGGTCTTTCGCATCCTGTGGCTCTTACCTTCACCAAGGAGAATT
CTCTTCTTGTGCTGGACACAGCATCTCATTCTATAAAAGTCTATAAAGTTGA
CTGGGGGTGAgcggccgcaaGAATTCgatatcaagcttatcgataatcaacctctggattacaaaatttgtgaaagatt
gactggtattcttaactatgttgctccttttacgctatgtggatacgctgctttaatgcctttgtatcatgctattgcttcccgtatggctttcattt
tctcctccttgtataaatcctggttgctgtctctttatgaggagttgtggcccgttgtcaggcaacgtggcgtggtgtgcactgtgtttgctg

acgcaaccccactggttggggcattgccaccacctgtcagctcctttccgggactttcgctttccccctccctattgccacggcggaac
tcatcgccgcctgccttgcccgctgctggacaggggctcggctgttgggcactgacaattccgtggtgttgtcggggaaATCATc
gtcctttccTtggctgctcgcctgtgttgccacctggattctgcgcgggacgtccttctgctacgtcccttcggccctcaatccagcgga
ccttccttcccgcggcctgctgccggctctgcggcctcttccgcgtcttcgccttcgccctcagacgagtcggatctccctttgggccgc
ctccccgcctgatcgataccgtcgacctcgagggggggcccggtaccCGGGAATCAATTCACTCCTCAGGTG
CAGGCTGCCTATCAGAAGGTGGTGGCTGGTGTGGCCAATGCCCTGGCTCACAAA
TACCACTGAGATCTTTTTCCCTCTGCCAAAAATTATGGGGACATCATGAAGCCCC
TTGAGCATCTGACTTCTGGCTAATAAAGGAAATTTATTTTCATTGCAATAGTGTGT
TGGAATTTTTTGTGTCTCTCACTCGGAAGGACATATGGGAGGGCAAATCATTTAA
AACATCAGAATGAGTATTTGGTTTAGAGTTTGGCAACATATGCCCATATGCTGGC
TGCCATGAACAAAGGTTGGCTATAAAGAGGTCATCAGTATATGAAACAGCCCCC
TGCTGTCCATTCCTTATTCCATAGAAAAGCCTTGACTTGAGGTTAGATTTTTTTA
TATTTTGTTTTGTGTTATTTTTTTCTTTAACATCCCTAAAATTTTCCTTACATGTTTT
ACTAGCCAGATTTTTCCTCCTCTCCTGACTACTCCCAGTCATAGCTGTCCCTCTTC
TCTTATGGAGATCCCTCGACCTGCAGCCCAAGCTGTAGATAAGTAGCATGGCGG
GTTAATCATTAACTACAAGGAACCCCTAGTGATGGAGTTGGCCACTCCCTCTCTG
CGCGCTCGCTCGCTCACTGAGGCCGGGCGACCAAAGGTCGCCCGACGCCCGGGC
TTTGCCCGGGCGGCCTCAGTGAGCGAGCGAGCGCGCAGCTGGCGTAATAGCGAA
GAGGCCCGCACCGATCGCCCTTCCCAACAGTTGCGCAGCCTGAATGGCGAATGG
CGATTCCGTTGCAATGGCTGGCGGTAATATTGTTCTGGATATTACCAGCAAGGCC
GATAGTTTGAGTTCTTCTACTCAGGCAAGTGATGTTATTACTAATCAAAGAAGTA
TTGCGACAACGGTTAATTTGCGTGATGGACAGACTCTTTTACTCGGTGGCCTCAC
TGATTATAAAAACACTTCTCAGGATTCTGGCGTACCGTTCCTGTCTAAAATCCCTT
TAATCGGCCTCCTGTTTAGCTCCCGCTCTGATTCTAACGAGGAAAGCACGTTATA
CGTGCTCGTCAAAGCAACCATAGTACGCGCCCTGTAGCGGCGCATTAAGCGCGG
CGGGTGTGGTGGTTACGCGCAGCGTGACCGCTACACTTGCCAGCGCCCTAGCGCC
CGCTCCTTTCGCTTTCTTCCCTTCCTTTCTCGCCACGTTCGCCGGCTTTCCCCGTCA
AGCTCTAAATCGGGGGCTCCCTTTAGGGTTCCGATTTAGTGCTTTACGGCACCTC
GACCCCAAAAAACTTGATTAGGGTGATGGTTCACGTAGTGGGCCATCGCCCTGAT
AGACGGTTTTTCGCCCTTTGACGTTGGAGTCCACGTTCTTTAATAGTGGACTCTTG
TTCCAAACTGGAACAACACTCAACCCTATCTCGGTCTATTCTTTTGATTTATAAGG
GATTTTGCCGATTTCGGCCTATTGGTTAAAAAATGAGCTGATTTAACAAAAATTT
AACGCGAATTTTAACAAAATATTAACGCTTACAATTTAAATATTTGCTTATACAA
TCTTCCTGTTTTTGGGGCTTTTCTGATTATCAACCGGGGTACATATGATTGACATG

CTAGTTTTACGATTACCGTTCATCGATTCTCTTGTTTGCTCCAGACTCTCAGGCAA
TGACCTGATAGCCTTTGTAGAGACCTCTCAAAAATAGCTACCCTCTCCGGCATGA
ATTTATCAGCTAGAACGGTTGAATATCATATTGATGGTGATTTGACTGTCTCCGG
CCTTTCTCACCCGTTTGAATCTTTACCTACACATTACTCAGGCATTGCATTTAAAA
TATATGAGGGTTCTAAAAATTTTTATCCTTGCGTTGAAATAAAGGCTTCTCCCGC
AAAAGTATTACAGGGTCATAATGTTTTTGGTACAACCGATTTAGCTTTATGCTCT
GAGGCTTTATTGCTTAATTTTGCTAATTCTTTGCCTTGCCTGTATGATTTATTGGAT
GTTGGAATCGCCTGATGCGGTATTTTCTCCTTACGCATCTGTGCGGTATTTCACAC
CGCATATGGTGCACTCTCAGTACAATCTGCTCTGATGCCGCATAGTTAAGCCAGC
CCCGACACCCGCCAACACCCGCTGACGCGCCCTGACGGGCTTGTCTGCTCCCGGC
ATCCGCTTACAGACAAGCTGTGACCGTCTCCGGGAGCTGCATGTGTCAGAGGTTT
TCACCGTCATCACCGAAACGCGCGAGACGAAAGGGCCTCGTGATACGCCTATTTT
TATAGGTTAATGTCATGATAATAATGGTTTCTTAGACGTCAGGTGGCACTTTTCG
GGGAAATGTGCGCGGAACCCCTATTTGTTTATTTTTCTAAATACATTCAAATATGT
ATCCGCTCATGAGACAATAACCCTGATAAATGCTTCAATAATATTGAAAAAGGA
AGAGTATGAGTATTCAACATTTCCGTGTCGCCCTTATTCCCTTTTTTGCGGCATTT
TGCCTTCCTGTTTTTGCTCACCCAGAAACGCTGGTGAAAGTAAAAGATGCTGAAG
ATCAGTTGGGTGCACGAGTGGGTTACATCGAACTGGATCTCAACAGCGGTAAGA
TCCTTGAGAGTTTTCGCCCCGAAGAACGTTTTCCAATGATGAGCACTTTTAAAGT
TCTGCTATGTGGCGCGGTATTATCCCGTATTGACGCCGGGCAAGAGCAACTCGGT
CGCCGCATACACTATTCTCAGAATGACTTGGTTGAGTACTCACCAGTCACAGAAA
AGCATCTTACGGATGGCATGACAGTAAGAGAATTATGCAGTGCTGCCATAACCA
TGAGTGATAACACTGCGGCCAACTTACTTCTGACAACGATCGGAGGACCGAAGG
AGCTAACCGCTTTTTTGCACAACATGGGGGATCATGTAACTCGCCTTGATCGTTG
GGAACCGGAGCTGAATGAAGCCATACCAAACGACGAGCGTGACACCACGATGCC
TGTAGCAATGGCAACAACGTTGCGCAAACTATTAACTGGCGAACTACTTACTCTA
GCTTCCCGGCAACAATTAATAGACTGGATGGAGGCGGATAAAGTTGCAGGACCA
CTTCTGCGCTCGGCCCTTCCGGCTGGCTGGTTTATTGCTGATAAATCTGGAGCCG
GTGAGCGTGGGTCTCGCGGTATCATTGCAGCACTGGGGCCAGATGGTAAGCCCT
CCCGTATCGTAGTTATCTACACGACGGGGAGTCAGGCAACTATGGATGAACGAA
ATAGACAGATCGCTGAGATAGGTGCCTCACTGATTAAGCATTGGTAACTGTCAG
ACCAAGTTTACTCATATATACTTTAGATTGATTTAAAACTTCATTTTTAATTTAAA
AGGATCTAGGTGAAGATCCTTTTTGATAATCTCATGACCAAAATCCCTTAACGTG
AGTTTTCGTTCCACTGAGCGTCAGACCCCGTAGAAAAGATCAAAGGATCTTCTTG

AGATCCTTTTTTTCTGCGCGTAATCTGCTGCTTGCAAACAAAAAAACCACCGCTA
CCAGCGGTGGTTTGTTTGCCGGATCAAGAGCTACCAACTCTTTTTCCGAAGGTAA
CTGGCTTCAGCAGAGCGCAGATACCAAATACTGTTCTTCTAGTGTAGCCGTAGTT
AGGCCACCACTTCAAGAACTCTGTAGCACCGCCTACATACCTCGCTCTGCTAATC
CTGTTACCAGTGGCTGCTGCCAGTGGCGATAAGTCGTGTCTTACCGGGTTGGACT
CAAGACGATAGTTACCGGATAAGGCGCAGCGGTCGGGCTGAACGGGGGGTTCGT
GCACACAGCCCAGCTTGGAGCGAACGACCTACACCGAACTGAGATACCTACAGC
GTGAGCTATGAGAAAGCGCCACGCTTCCCGAAGGGAGAAAGGCGGACAGGTATC
CGGTAAGCGGCAGGGTCGGAACAGGAGAGCGCACGAGGGAGCTTCCAGGGGGA
AACGCCTGGTATCTTTATAGTCCTGTCGGGTTTCGCCACCTCTGACTTGAGCGTCG
ATTTTTGTGATGCTCGTCAGGGGGGCGGAGCCTATGGAAAAACGCCAGCAACGC
GGCCTTTTTACGGTTCCTGGCCTTTTGCTGGCCTTTTGCTCACATGTTCTTTCCTGC
GTTATCCCCTGATTCTGTGGATAACCGTATTACCGCCTTTGAGTGAGCTGATACC
GCTCGCCGCAGCCGAACGACCGAGCGCAGCGAGTCAGTGAGCGAGGAAGCGGA
AGAGCGCCCAATACGCAAACCGCCTCTCCCCGCGCGTTGGCCGATTCATTAATGC
AGCTGCGCGCTCGCTCGCTCACTGAGGCC.

In a preferred embodiment, the nucleic acid sequence encoding EPM2B is operably linked to a regulatory sequence that drives the expression of the coding sequence.

In a preferred embodiment, the vector is a non-integrative vector.

In a preferred embodiment, the vector is an adeno-associated virus-based non-integrative vector, preferably derived from a serotype 2 or 9 adeno-associated virus.

In a preferred embodiment, the capsid of the adeno-associated virus-based vector is made of a capsid proteins of the serotype 9 adeno-associated virus, and the nucleic acid sequence contained in the capsid is flanked at both ends by internal terminal repeats corresponding to serotype 2 adeno-associated virus.

In a preferred embodiment, the vector comprises a regulatory sequence which is a constitutive promoter, preferably CAG.

The present invention also refers to the vector of the invention for use a as a medicament, that comprises the administration of a therapeutically effective dose or amount of the vector of the invention or a composition comprising thereof. Alternatively, the present invention refers to a method for the treatment of a disease or medical condition caused by the deletion Epm2b-/-, such as Lafora disease, which comprises the administration of a therapeutically effective dose or amount of the vector of the invention, optionally in combination with pharmaceutically acceptable excipients or carriers.

In a preferred embodiment, the present invention refers to the vector of the invention for use in a method for the treatment of Lafora disease.

In a preferred embodiment, the present invention refers to the vector of the invention for use in a method for the treatment of Lafora disease, wherein the method comprises the administration of the vector to a presymptomatic subject diagnosed with or predisposed to Lafora disease.

In a preferred embodiment the route of administration may be selected from: intracerebroventricular, intrathecal or intravenous administration.

For the purpose of the present invention the following terms are defined:
- "Pharmaceutically acceptable excipient or carrier" refers to an excipient that may optionally be included along with the vector of the invention and that causes no significant adverse toxicological effects to the patient.
- By "therapeutically effective dose or amount" of the vector of the invention, the present invention refers to the situation when the vector of the invention is administered and brings about a positive therapeutic response in a subject. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.
- A "presymptomatic subject" is an individual carrying a pathogenic mutation in the EPM2A or EPM2B gene, identified through genetic screening or biomarker analysis, who has not yet exhibited clinical symptoms of Lafora disease; and
- A "predisposed subject" is an individual at high risk of developing Lafora disease due to genetic inheritance, family history, or early detectable pathological changes, as determined by genetic counseling, molecular diagnostics, or biochemical markers.

### Brief description of the figures

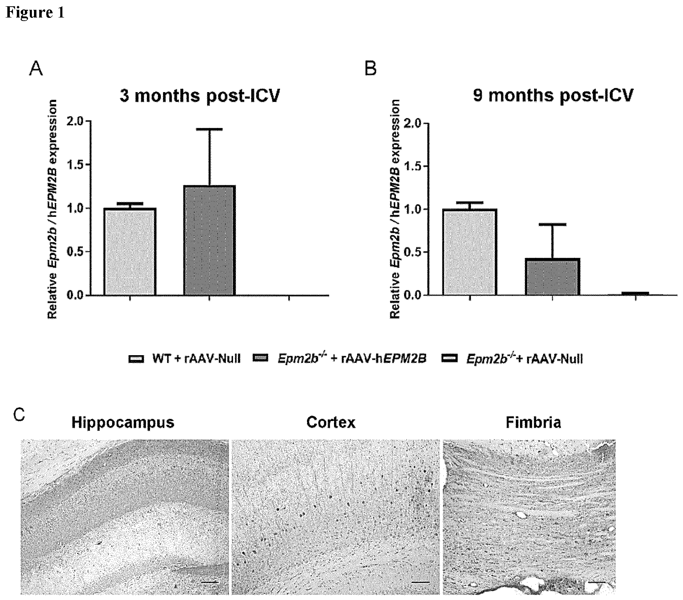

**Figure 1****. Analysis of *hEPM2B* transcription and GFP expression in the brain 3 and 9 months after a single ICV injection of the rAAV*-*h*EPM2B* or rAAV-*GFP* vectors. (A, B)** RT-qPCR quantification of *Epm2b* cDNA levels in the brains of WT, and *hEPM2B* cDNA levels in the brains of *Epm2b*^{*-*/*-*} mice, 3 **(A)** and 9 **(B)** months post-injection, normalized to housekeeping *Gapdh* levels. **(C)** Biodistribution of the rAAV2/9 vector in the brain of WT mice 3 months after ICV injection of the rAAV-*GFP* vector, analyzed by immunohistochemistry with a GFP antibody. Left panel, hippocampus; central panel, cortex; right panel, fimbria. Scale bar=100 µm.

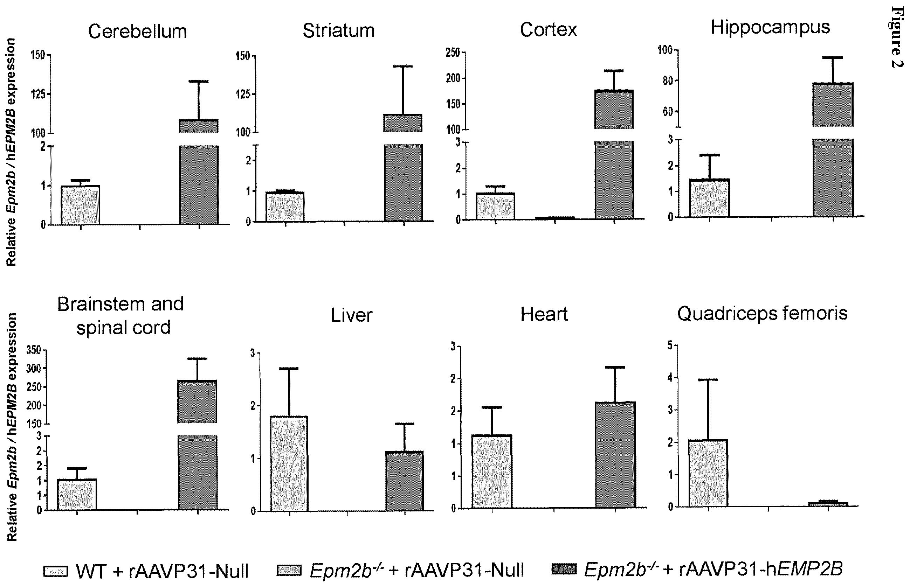

**Figure 2****. RT-qPCR quantification of human *EPM2B* cDNA in different areas of the CNS and in the heart, liver and quadriceps femoris, 5 months after a single IV injection of the rAAVP31-h*EPM2B* vectors *Epm2b*^{*-*/*-*} mice.** RT-qPCR quantification showing the levels of *EPM2B* cDNA in different regions of the CNS, as well as in the heart, liver and quadriceps femoris samples collected from WT, and *Epm2b*^{*-*/*-*} mice, 5 months after IV injection of *rAAVP31-hEPM2B* or rAAVP31-Null vectors. The gene expression levels were normalized to the values observed for the *Gadph* gene. Data are shown as mean ±SEM. n= 3-4 mice per group and experiment.

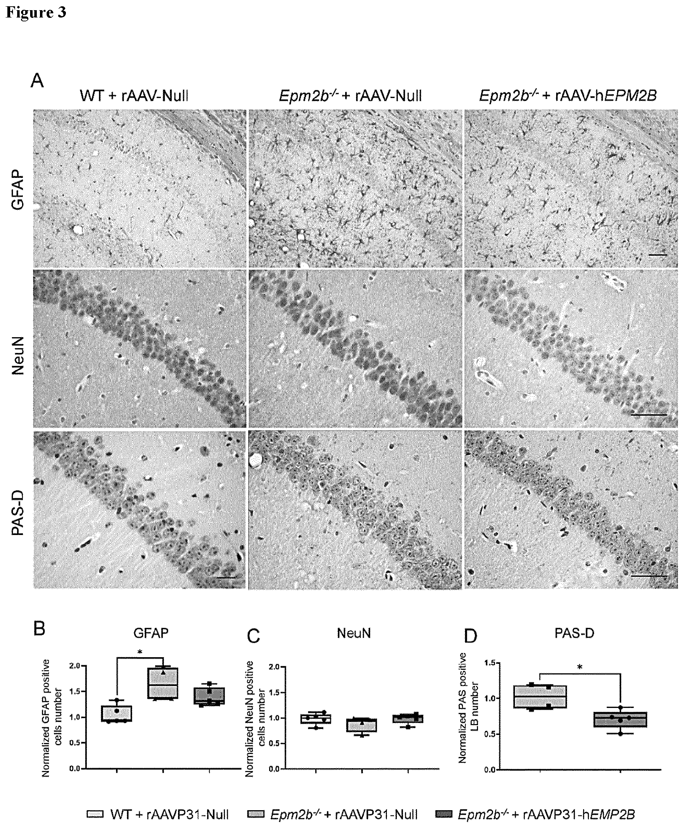

**Figure 3****. Quantification of GFAP- and NeuN- positive cells, and LB formation in the CA1 region of the hippocampus of *Epm2b*^{*-*/*-*} mice IV injected with rAAVP31-h*EPM2B*. (A)** IHC was performed with an anti-GFAP antibody to stain reactive astrocytes, with NeuN antibody labeling neuronal cells, and PAS-D staining to visualize LBs in WT and *Epm2b*^{*-*/*-*} 6-month-old mice, 5 months after *rAAVP31-hEPM2B* or rAAVP31-Null treatment. Scale bar = 50 µm. **(B)** Quantification of reactive astrocytes. Data are shown as the median of independent samples, with normalization based on WT mice treated with rAAVP31-Null values. The bars in the box plots show the minimum and maximum values, Statistical analysis was performed using a non-parametric Kruskal-Wallis test followed by Dunn's multiple comparisons. * p < 0.05. n = 4-5 mice per group. **(C)** Quantification of NeuN-positive cells. Data are shown as the median of independent samples. Boxplot bars indicate the minimum and maximum values. Values were normalized using WT mice treated with rAAVP31-Null values. Statistical analysis was conducted using a non-parametric Kruskal-Wallis test followed by Dunn's multiple comparisons. * p < 0.05. n = 4-5 mice per group. **(D)** Quantitative comparison of LB numbers. Data are shown as the median of independent samples. Boxplot bars represent the minimum and maximum values. Values were normalized using *Epm2b*^{*-*/*-*} mice treated with rAAVP31-Null values. Statistical analysis was performed using a non-parametric Mann-Whitney test. * p < 0.05. n = 4-6 mice per group.

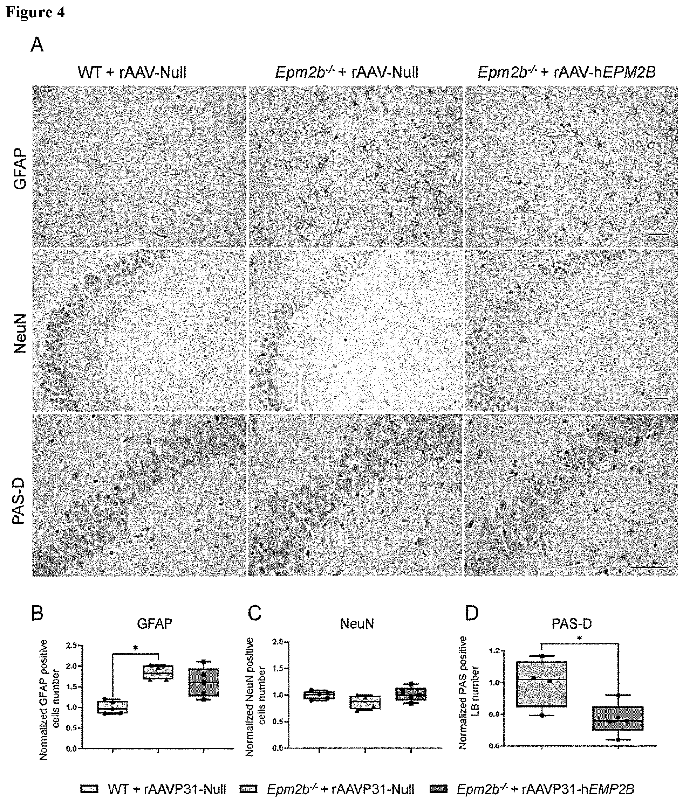

Figure **4****. Quantification of GFAP- and NeuN-positive cells, and LB formation in the CA2-CA3 region of the hippocampus of 6-month-old *Epm2b*^{*-*/*-*} mice IV injected with rAAVP31-h*EPM2B*. (A)** IHC was performed with an anti-GFAP antibody to stain reactive astrocytes, with NeuN antibody to label neuronal cells, and PAS-D staining to visualize LBs in WT and *Epm2b*^{*-*/*-*} mice 5 months after *rAAVP31-hEPM2B* or rAAVP31-Null treatment. Scale bar = 50 µm. **(B)** Quantification of reactive astrocytes. Data are shown as the median of independent samples, normalized to WT mice treated with rAAVP31-Null values. The bars in the box plots show the minimum and maximum values. Statistical analysis was performed using a non-parametric Kruskal-Wallis test followed by Dunn's multiple comparisons. * p < 0.05. n = 4-5 mice per group. **(C)** Quantification of NeuN-positive cells. Data are shown as the median of independent samples. The bars in the box plots show the minimum and maximum values. Values were normalized using WT mice treated with rAAVP31-Null values. A non-parametric Kruskal-Wallis test was performed followed by Dunn's multiple comparisons. * p < 0.05. n = 4-5 mice per group. **(D)** Quantitative comparison of LB numbers. Data are shown as the median of independent samples. The bars in the box plots show the minimum and maximum values. Values were normalized using *Epm2b*^{*-*/*-*} mice treated with rAAVP31-Null values. Statistical analysis was performed using a non-parametric Mann-Whitney test. * p < 0.05. n = 4-6 mice per group.

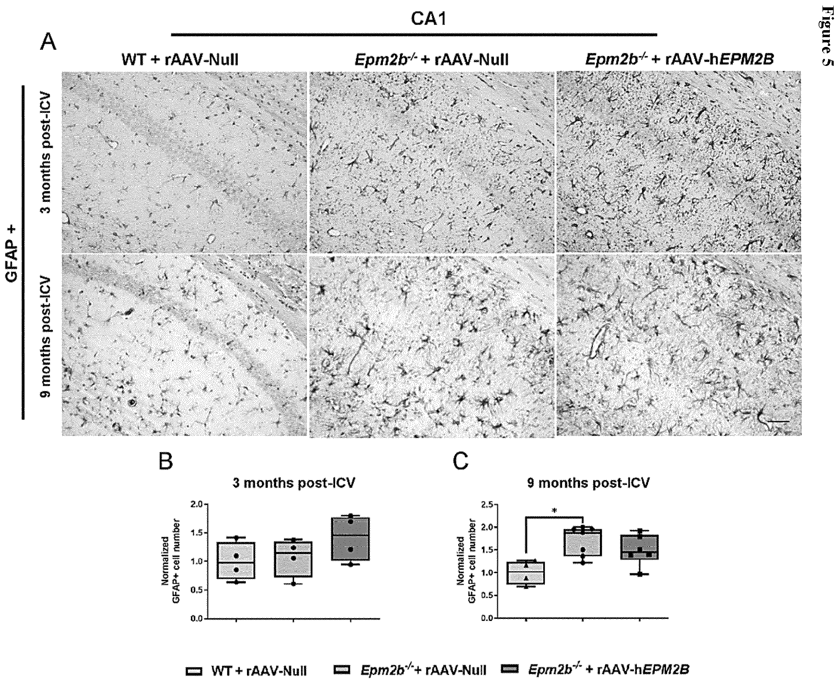

**Figure 5****. Analysis of astrogliosis in the CA1 region of the hippocampus in *Epm2b*^{*-*/*-*} mice 3 and 9 months post-injection. (A)** Representative images of GFAP immunostaining in the CA1 field of the hippocampus 3 and 9 months after rAAV-h*EPM2B* or rAAV-Null administration in *Epm2b*^{*-*/*-*} mice. **(B, C)** Quantification of GFAP-positive cells expressed as the median of independent samples. Boxplot bars indicate minimum and maximum values. Data were normalized to levels observed in WT mice injected with rAAV-Null. Statistical analysis was conducted using a non-parametric Kruskal-Wallis test followed by Dunn's multiple comparisons. * p < 0.05. n = 4-6 mice per group. Scale bar = 50 µm.

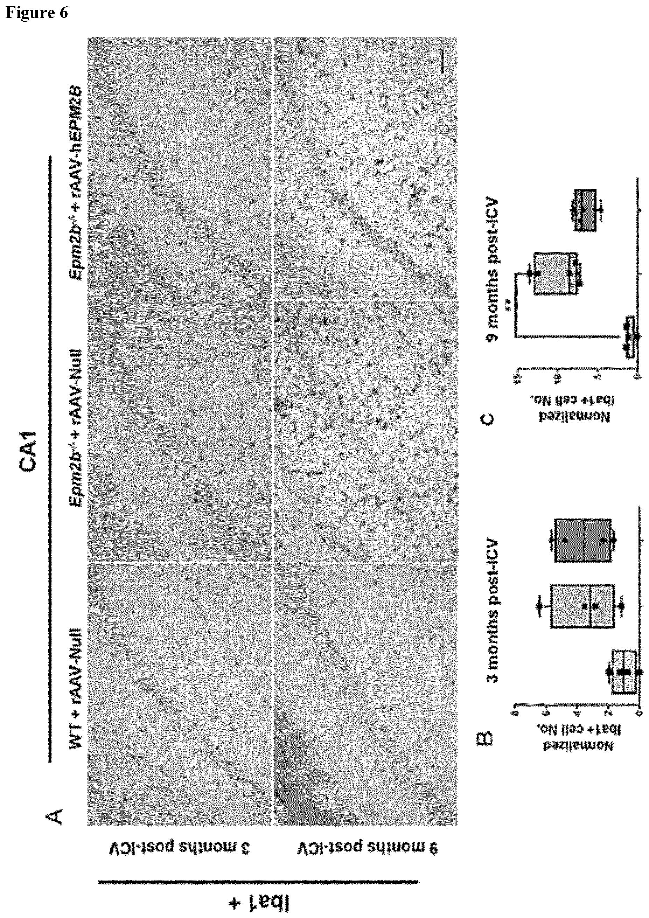

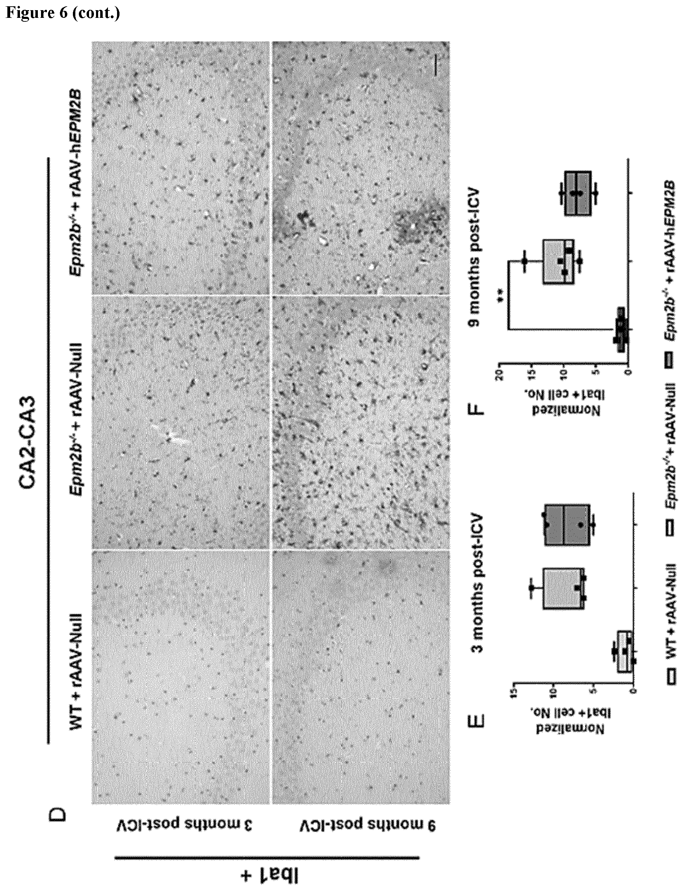

**Figure 6****. Microgliosis in the hippocampus of *Epm2b*^{*-*/*-*} mice (A, D)** IHC with an anti-Iba1 antibody to localize microglia. **(B, C, E, F)** Quantitative analysis of microgliosis in the CA1 **(B, C)** and CA2-CA3 **(E, F)** regions of the hippocampus of WT mice injected with rAAV-Null and *Epm2b*^{*-*/*-*} mice treated with rAAV-h*EPM2B* or rAAV-Null, 3 months **(B, E)** and 9 months **(C, F)** post-treatment. Results are expressed as the median of independent samples, with bars in the boxplots representing minimum and maximum values. Values were normalized to those of WT mice injected with rAAV-Null. Statistical analysis was performed using a non-parametric Kruskal-Wallis test followed by Dunn's multiple comparisons test. * p < 0.05, ** p < 0.01. n = 4-5 mice per group. Scale bars = 50 µm.

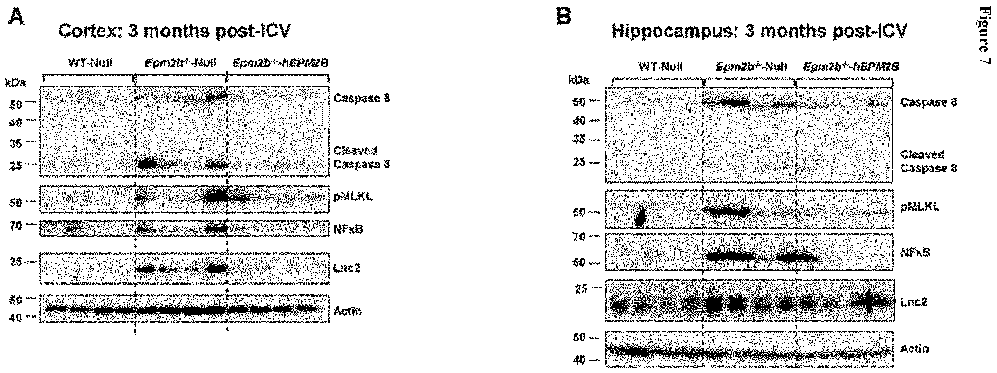

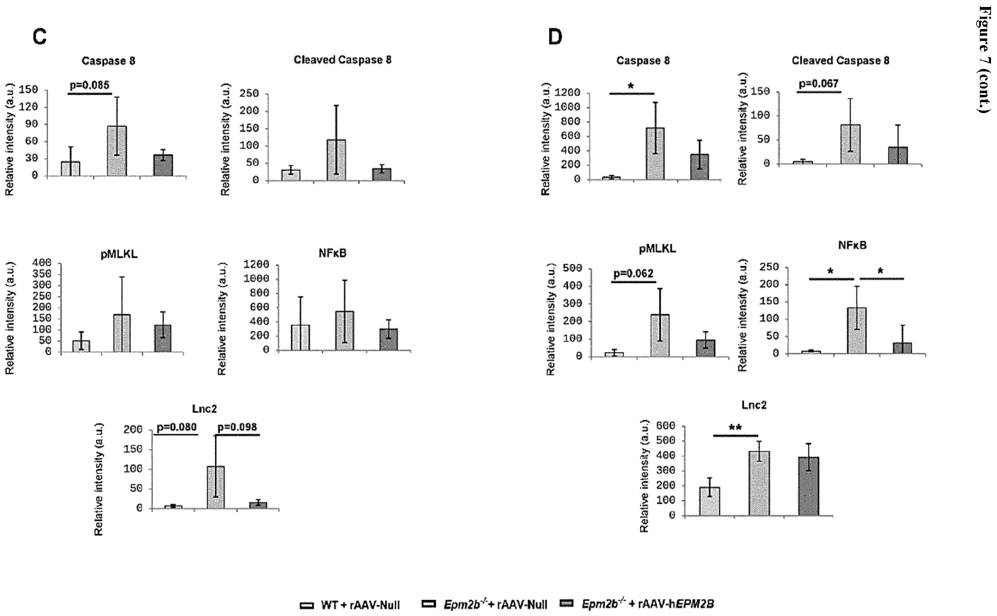

**Figure 7****. Levels of neuroinflammatory markers in the cortex and hippocampus 3 months post-ICV injection of rAAV-Null or rAAV*-*h*EPM2B* in WT and *Epm2b*^{*-*/*-*} mice** Protein levels of caspase-8 (full length and cleaved), phospho (Ser345)-MLKL (pMLKL), NFκB, lipocalin 2 (Lnc2), and actin were assessed by western blot in cortical **(A)** and hippocampal extracts **(B)** from WT mice injected with rAAV-Null and *Epm2b*^{*-*/*-*} mice treated with either rAAV-h*EPM2B* or rAAV-Null 3 months post-ICV injection. Molecular weight standards are shown on the left. Densitometric quantification of the blots was carried out as described in the Materials and Methods section, with values normalized to actin and represented as arbitrary units (a.u.) for the cortex **(C)** and hippocampus **(D).** Four independent samples from each genotype were analyzed. Results are expressed as means +/standard deviation (SD). Differences between paired samples were analyzed by two-tailed Student's t-tests using Graph Pad Prism version 5.0 statistical software (La Jolla, CA, USA). Statistical significance was considered at *p<0.05 and **p< 0.01.

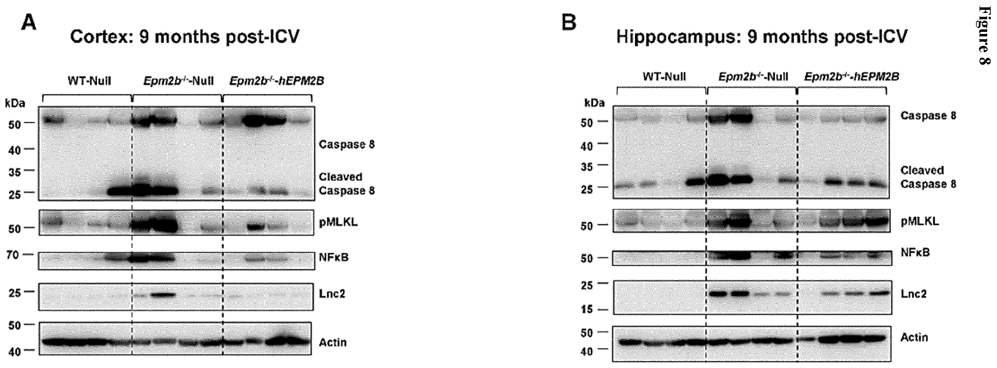

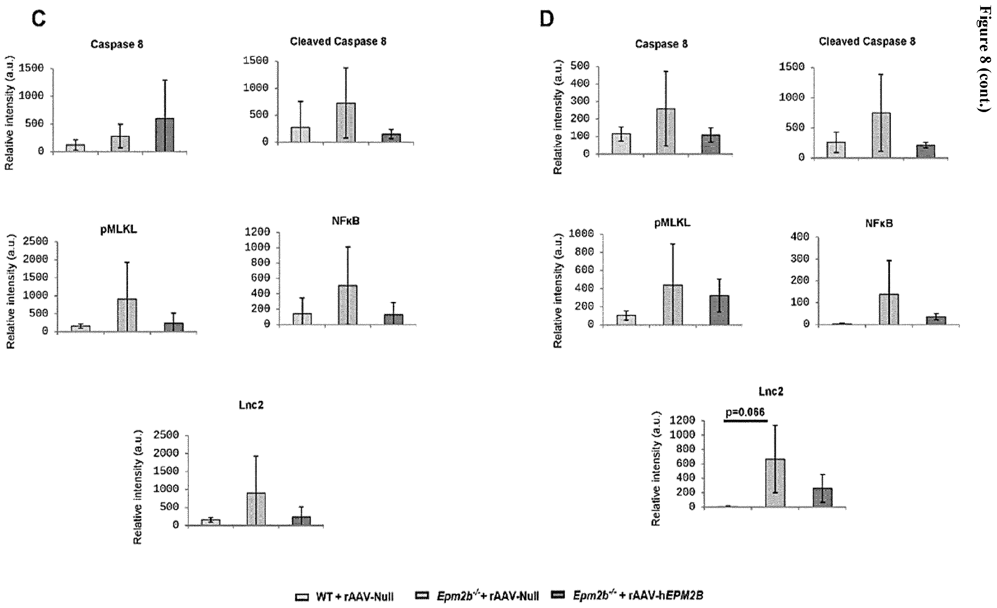

**Figure 8****. Levels of neuroinflammatory markers in the cortex and hippocampus 9 months post-ICV injection of rAAV-Null or rAAV*-*h*EPM2B* in WT and *Epm2b*^{*-*/*-*} mice** Protein levels of caspase-8 (full length and cleaved), phospho (Ser345)-MLKL (pMLKL), NFκB, lipocalin 2 (Lnc2) and actin were assessed by western blot of cortical **(A)** and hippocampal extracts **(B)** from WT mice injected with rAAV-Null and *Epm2b*^{*-*/*-*} mice treated with either rAAV-h*EPM2B* or rAAV-Null, 9 months post-ICV injection. Molecular weight markers are shown on the left. Densitometric quantification of the blots was performed as described in the Materials and Methods section, with values normalized to actin and represented as arbitrary units (a.u.) for the cortex **(C)** and hippocampus **(D).** Four independent samples from each genotype were analyzed. Results are expressed as means +/standard deviation (SD). Differences between paired samples were analyzed by two-tailed Student's t-tests using Graph Pad Prism version 5.0 statistical software (La Jolla, CA, USA).

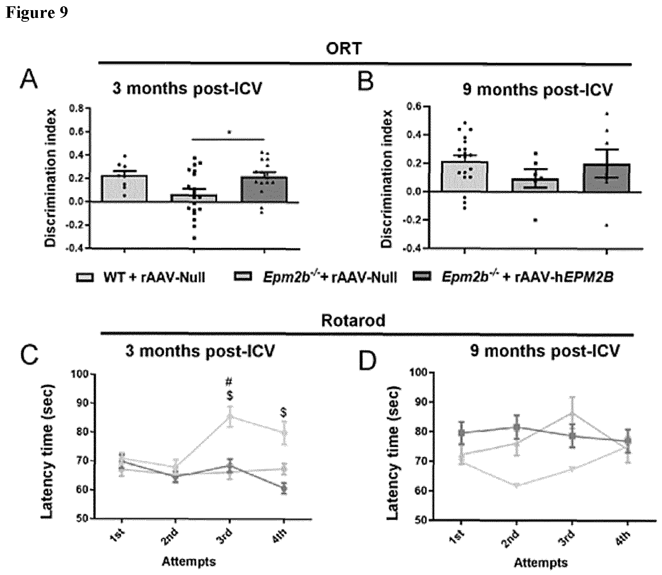

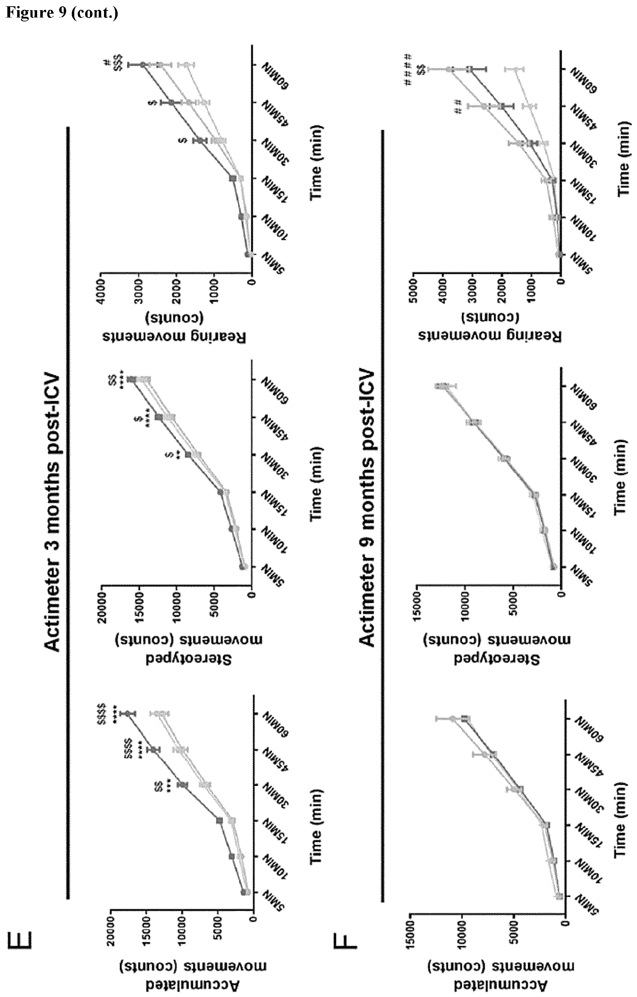

**Figure 9****. Behavioral assessments in *Epm2b*^{*-*/*-*} mice treated with rAAV-h*EPM2B,* and in *Epm2b*^{*-*/*-*} and WT mice injected with rAAV-Null. (A, B)** Memory performance based on the D.I. obtained from the ORT, 3 (A) and 9 (B) months post-ICV injection. **(C, D)** Evaluation of motor coordination based on the latency to fall from the cylinder in the rotarod test, 3 **(C)** and 9 **(D)** months post-ICV treatment (# WT + rAAV-Null vs. *Epm2b*^{*-*/*-*} + rAAV-Null; $ WT + rAAV-Null vs. *Epm2b*^{*-*/*-*} + rAAV-h*EPM2B*). **(E, F)** Analysis of spontaneous accumulated, stereotyped and rearing movements recorded over one hour in the actimeter, 3 **(E)** and 9 **(F)** months after ICV injection *(* Epm2b*^{*-*/*-*} + rAAV-h*EPM2B* vs. *Epm2b*^{*-*/*-*} + rAAV-Null; # WT + rAAV-Null vs. *Epm2b*^{*-*/*-*} + rAAV-Null; $ WT + rAAV-Null vs. *Epm2b*^{*-*/}*⁻ +* rAAV-hEPM2B). Data are shown as mean ± SEM. Statistical comparison between groups were conducted using one- and two-way ANOVA with Tukey's multiple comparisons tests. * p < 0.05, ** p < 0.01, *** p <0.001, **** p < 0.0001. n = 15-25 mice per group per experiment.

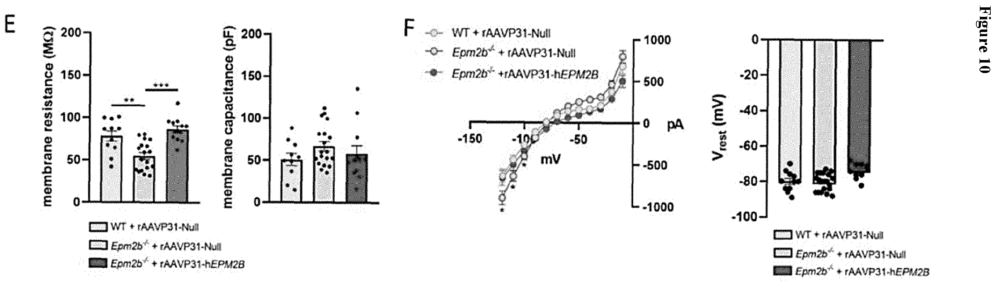

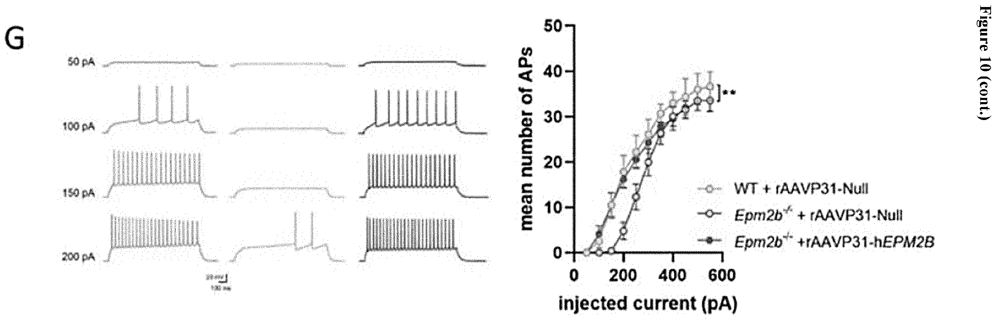

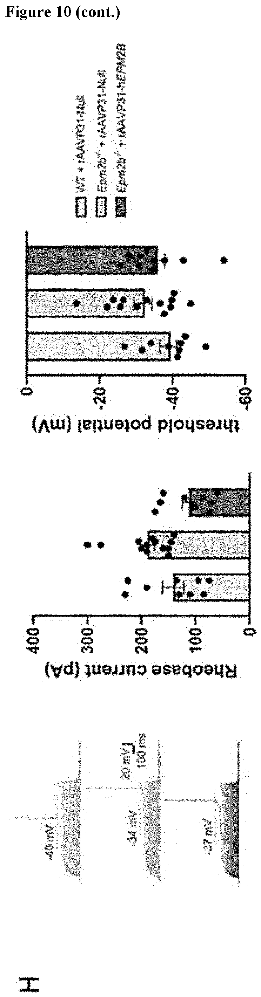

**Figure 10****. Basal membrane properties and action potential discharges of striatal MSNs in WT and *Epm2b*^{*-*/*-*} mice after IV injection of rAAVP31-Null or rAAVP31-h*EPM2B*** vectors. **(E)** Left panel: histograms of membrane resistance showing a significant reduction in MSNs of *Epm2b*^{*-*/*-*} +rAA VP31-Null relative to WT. Note that IV injection with rAAVP31-*hEPM2B* vector completely restored membrane resistance to control values (Rm: WT=78.21±6 MΩ, n=11; *Epm2b*^{*-*/}*⁻+*rAAVP31-Null*=* 54.88±4 MΩ, n=18; *Epm2b*^{-/-}+rAAVP31-h*EPM2B*=86.54±4.1 MΩ, n=12; **p<0.01, ***p<0.001 Student's t-test); right panel: histograms of MSNs membrane capacitance showing no differences between the three groups (Cm: WT= 50.94±7.3 pF, n=10; *Epm2b*^{*-*/*-*} +rAAVP31-Null= 66.87±5.6 pF, n=19; *Epm2b*^{*-*/*-*}+rAAVP31-h*EPM2B*= 58.06±7.3 pF, n=12). **(F)** Mean current-voltage plot (left) recorded from MSNs of WT, *Epm2b*^{-/-}+rAAVP31-Null and *Epm2b*^{*-*/}*⁻+*rAAVP31-h*EPM2B* mice during injection of hyperpolarizing and depolarizing current steps, showing a significant increase of the inward currents elicited at hyperpolarized membrane potential in *Epm2b*^{*-*/-}compared to WT, which is completely restored after IV injection of *Epm2b*^{*-*/}*⁻+*rAAVP31-*hEPM2B* vector **(-120mV:** WT= -638.9±81.6 pA, n=12; *Epm2b*^{*-*/}*⁻+*rAAVP31-Null*=* - 902.0±82.9 pA, n=21; *Epm2b*^{*-*/}*⁻+*rAAVP31-h*EPM2B*= -666.0±86.1 pA, n=11; **-110mV:** WT= -444.2±56.7 pA, n=12; *Epm2b*^{*-*/}*⁻+*rAAVP31-Null*=* -641.7±61.3 pA, n=21; *Epm2b*^{*-*/-}+rAAVP31-h*EPM2B*= -501.9±70.8 pA, n=11; **-100mV:** WT= -265.4±33.2 pA, n=12; *Epm2b*^{*-*/*-*}+rAAVP31-Null*=* -400.5±42.8 pA, n=21; *Epm2b*^{*-*/*-*}+rAAVP31*-h*EPM2B*= -* 342.3±54.8 pA, n=11; WT vs *Epm2b*^{-/-}+rAAVP31-Null *p<0.05, Student's t-test). On the right, histograms of resting membrane potential showing no differences between the three groups (WT= -80.0±1.7 mV, n=11; *Epm2b*^{*-*/}*⁻+*rAAVP31-Null*=* -80.3±1.1 mV, n=20; *Epm2b*^{*-*/-}+rAAVP31-hEPM2B= -73.7±1.4 mV, n=10). (G) Action potential (AP) firing patterns in response to 50 pA-stepped depolarizing current injections (1200 msec duration) from MSNs in WT and *Epm2b*^{*-*/*-*} mice injected with either rAAVP31-Null or rAAVP31*-*h*EPM2B* vectors. The plot shows mean (± SEM) number of MSN APs at the different input currents **(150pA:** WT= 10.45±2.9, n=11; *Epm2b*^{*-*/}*⁻+*rAAVP31-Null*=* 0.4±0.3, n=20; *Epm2b*^{*-*/*-*}+rAAVP31-*hEPM2B=* 10.56±2.6, n=9; **200pA:** WT= 17.82±3.6, n=11; *Epm2b*^{*-*/}*⁻+*rAAVP31-Null*=* 4.8±1.9, n=20; *Epm2b*^{*-*/*-*}+rAAVP31*-*h*EPM2B=* 16.22±1.8, n=9; ** p<0.01 WT vs *Epm2b*^{*-*/-}+rAA VP31-Null, Two Way Anova). **(H)** Example of current-clamp recordings (5 pA-stepped depolarizing current injections; 50 msec), scaled to show the AP threshold, in MSNs of WT, *Epm2b*^{*-*/}*⁻+*rAAVP31-Null and *Epm2b*^{*-*/}*⁻+*rAAVP31*-*h*EPM2B* mice. The plots show an increasing trend of rheobase current and a significant depolarized AP threshold in MSNs of *Epm2b*^{*-*/*-*} mice relative to WT **(IRheo:** WT= 141.7±19.8 pA, n=9; *Epm2b*^{*-*/*-*}+rAAVP31-Null*=* 189.2±13.5 pA, n=13; *Epm2b*^{*-*/}*⁻+*rAAVP31*-*h*EPM2B=*111.5±13.3 pA, n=10; **AP threshold:** WT= -38.8±2.3 mV, n=9; *Epm2b*^{*-*/}*⁻+*rAAVP31-Null*=* -31.78±2.5 mV, n=13; *Epm2b*^{*-*/-}+rAAVP31-h*EPM2B*= -35.28±2.6 pA, n=10). Data are reported as means ± SEM.

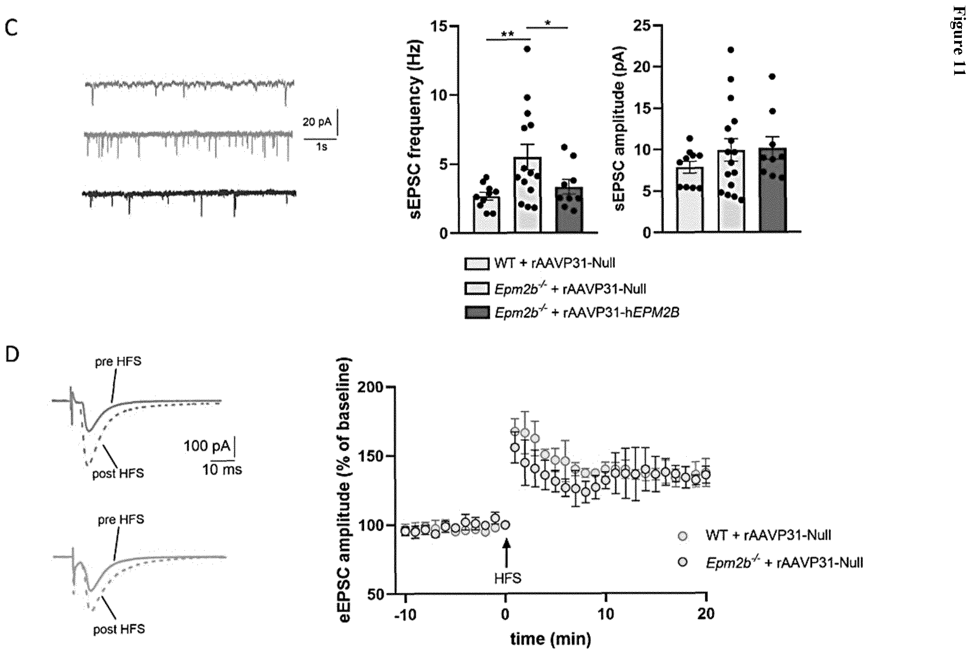

**Figure 11****. Cortico-striatal transmission and synaptic plasticity in the nucleus striatum of WTand *Epm2b*^{*-*/*-*} mice after IV injection of rAAVP31-h*EPM2B*, or rAAVP31-Null** vectors. (C) Representative sEPSC traces and histograms of the mean frequency and amplitude of the sEPSC of MSNs recorded from WT (green), *Epm2b*^{*-*/}*⁻+*rAAVP31-Null (light purple) and *Epm2b-*/-*+*rAAVP31-h*EPM2B* (purple) mice, showing a significant increase of sEPSC frequency and a trend toward reduction of sEPSC amplitude in *Epm2b*^{*-*/*-*} mice with respect to WT. Note the rescue of sEPSC frequency to control levels in mice injected with the rAAVP31-h*EPM2B* vector **(sEPSC frequency:** WT= 2.68±0.3 Hz,n=10; *Epm2b*^{*-*/-}+rAAVP31-Null= 5.50±0.9 Hz, n=14; *Epm2b*^{*-*/}*⁻+*rAAVP31*-*h*EPM2B=* 3.35±0.5 Hz, n=9; **sEPSC amplitude:** WT= 7.87±0.7 pA, n=10; *Epm2b*^{*-*/}*⁻+*rAAVP31-Null*=* 9.92±1.3 pA, n=14; *Epm2b*^{*-*/}*⁻+*rAAVP31-h*EPM2B=* 10.18±1.3 pA, n=9; *p<0.05, **p<0.01 Student's t-test). **(D)** Representative eEPSC traces and time course plot of eEPSC amplitudes recorded in the MSNs of dorsolateral striatum before and after HFS protocol administration on WT (green), *Epm2b*^{*-*/*-*}+rAAVP31-Null (light purple), showing no differences between the two groups (WT: 137±10.2%, n=6; *Epm2b*^{*-*/*-*} +rAAVP31-Null: 135±6.1%, n=5). Data are reported as means ± SEM.

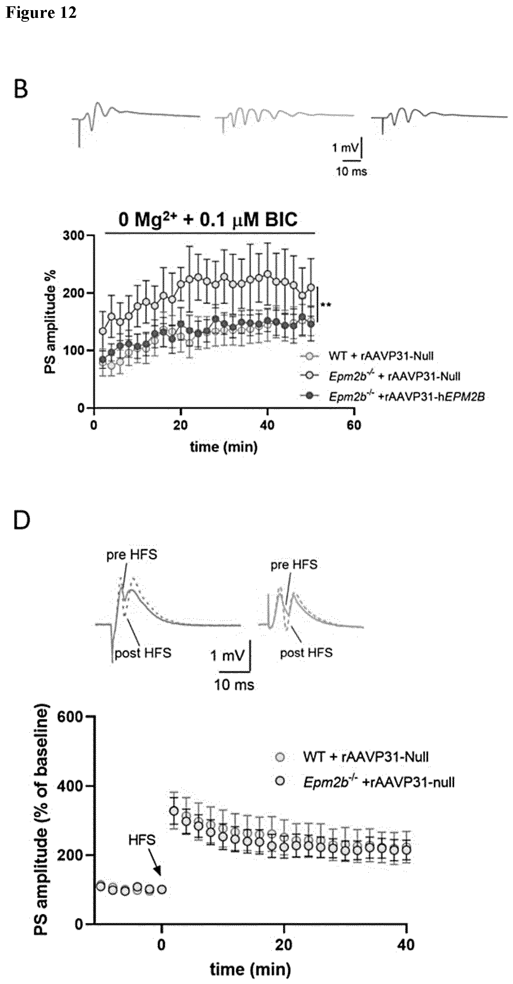

**Figure 12****. Epileptic-like activity and LTP in DG slices of WT and *Epm2b*^{*-*/*-*} mice after IV injection of rAAVP31-h*EPM2B* or rAAVP31-Null vectors. (B)** Representative traces of FPs and time-course graph of PS amplitude recorded in the DG of WT+rAA VP31-Null (green), *Epm2b^{-l-}+*rAAVP31*-*Null (light purple) and *Epm2b*^{*-*/*-*}+rAAVP31-h*EPM2B* (purple) mice in a magnesium-free ACSF in the presence of 0.1 µM bicuculline, showing a time-dependent increase of the epileptic-like activity in *Epm2b*^{-/-}+rAAV-Null mice relative to WT (WT: 152±26.3 %, n=16; *Epm2b*^{*-*/}*⁻+*rAAVP31-Null: 210±50.2 %, n=6; **p<0.01 two-way Anova). Injection of rAAVP31-h*EPM2B* vector reduces the epileptic-like activity (WT: 152±26.3 %, n=16; *Epm2b^{-l-}*+rAAVP31*-*h*EPM2B:* 146±29.7 %, n=10). **(D)** Representative PSs traces recorded before (left) and 40 min after (right) the HFS protocol in DG slices of WT (green) and *Epm2b*^{*-*/*-*}+rAAVP31-Null (light purple) mice. The time-course plot of PSs amplitudes recorded in the DG before and after HFS protocol shows no differences between the two groups (WT: 223±45.8 %, n=11; *Epm2b*^{*-*/}*⁻+*rAAVP31*-*Null: 215±28.7 %, n=5). Data are reported as means ± SEM.

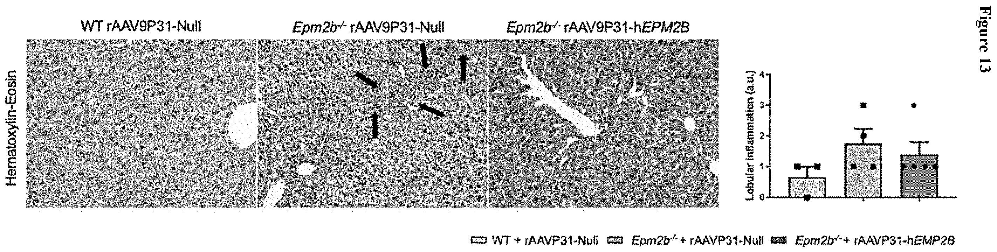

**Figure 13****. Hepatotoxicity study in 6-month-old WT and *Epm2b*^{*-*/*-*} mice, 5 months following IV administration of rAAVP31-h*EPM2B* or rAAVP31-Null vectors.** H&E staining in liver sections from WT and *Epm2b*^{*-*/*-*} mice, and semiquantitative analysis of lobular inflammation according to NASH activity scoring criteria. Abbreviations: a.u.= arbitrary units. n = 3-5 mice per group. Scale bar = 50 µm.

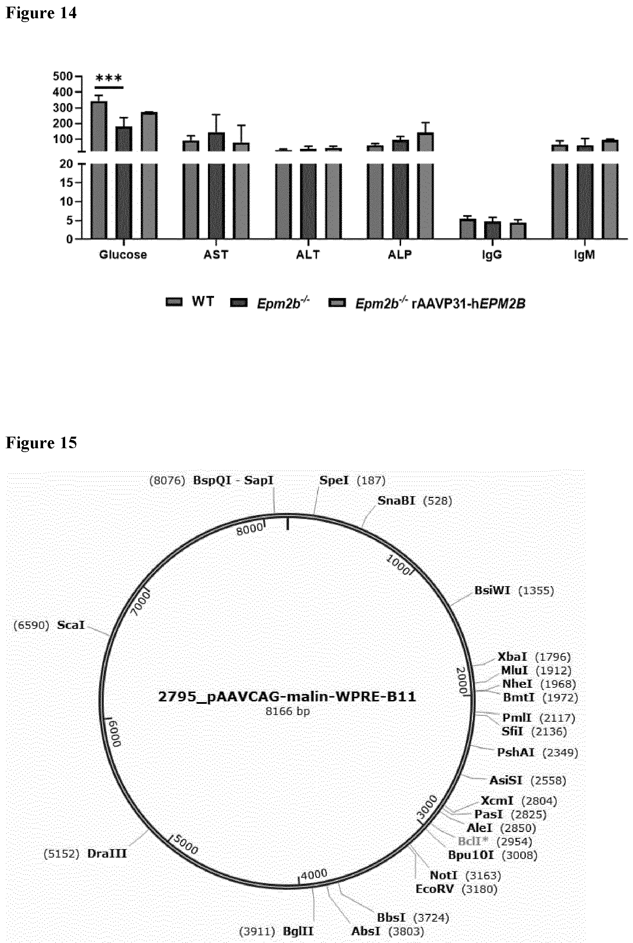

**Figure 14****. Serum liver and immune biochemical parameters in WT and *Epm2b*^{*-*/*-*} mice 16-19 months after IV injection.** Long-term effects of rAAVP31-h*EPM2B* IV administration on hepatic function and adaptative immune response. Data are shown as mean ± SEM. Statistical analysis was conducted using a two-way ANOVA with Tukey's multiple comparisons test. * p < 0.05. n= 2-4 mice per group.
**Figure 15****.** Plasmid map with restriction enzymes.

### EXAMPLES

The present invention is illustrated by means of the Examples set below, without the intention of limiting its scope of protection.

### Example 1. MATERIAL AND METHODS

### Example 1.1. Experimental Animals

The colonies of *Epm2b*^{*-*/*-*} and WT mice were housed in isolated cages within a controlled environment at the Animal Facility Service of the Instituto de Investigación Sanitaria-Fundación Jiménez Diaz. The conditions included a 12:12 light/dark cycle, a constant temperature of 23°C, and *ad libitum* access to food and water. Animal welfare was prioritized throughout the experiments, with efforts made to minimize distress and use and sacrifice the minimum number of animals necessary. Since no gender-related phenotypic differences have been described in mice or patients with LD, data from male and female mice were analyzed interchangeably. Procedures were carried out in accordance with the ethical guidelines established by the Instituto de Investigación Sanitaria-Fundación Jiménez Diaz Ethical Review Board, the European Communities Council Directive (2010/63/EU), and the "Principles of Laboratory Animal Care" (NIH publication No. 86-23, updated 1985).

### Example 1.2. Production of rAAV2/9-CAG-hEPM2B, rAAV2/9-CAG-Null and rAAV2/9-CAG-GFP vectors

The rAAV2/9-CAG-hEPM2B (rAAV-h*EPM2B*) vector, carrying the cDNA of the *hEPM2B* gene (NM_198586.3), the rAAV2/9-CAG-Null (rAAV-Null) vector, and the rAAV2/9-CAG-*GFP* (rAAV-GFP) vector were produced at the Unitat de Producció de Vectors (UPV_ www.viralvector.eu). These vectors were generated following the triple transfection system: (a) the ITR-containing plasmid; (b) the plasmid encoding the AAV capsid proteins (VP1, VP2, and VP3) and replicate genes; and (c) the adenoviral helper plasmid. AAV vectors were purified using iodixanol-based ultracentrifugation to remove empty capsids.

### Example 1.3. Stereotaxic ICV injections

Stereotaxic ICV injections were performed at 3 months of age. *Epm2b*^{*-*/*-*} and WT mice received a single ICV injection of either rAAV-h*EPM2B*, rAAV-Null, or rAAV-GFP vectors. To induce anesthesia, 4% isoflurane and 2% oxygen were administered, while 2% isoflurane and 1.5% oxygen were used to maintain it. The mice were secured in a stereotaxic frame (Stoelting, Illinois, USA). To ensure hydration, a subcutaneous saline injection (1 mL) was given, and ophthalmic gel was applied to prevent dry eyes. A heating pad was used to maintain a body temperature of 37°C. After sterilizing the area with 70% ethanol, a 2 cm incision was made behind the eyes. H₂O₂ was used to mark the bregma and lambda spots on the skull. A small burr hole was drilled using the coordinates of the right cerebral lateral ventricle (anteroposterior -0.3 mm, mediolateral -0.9 mm and dorsoventral -2.5 mm). Then, using a Hamilton^{®} syringe (ThermoFisher Scientific, Massachusetts, USA, Cat. #10664301), 3 µL of viral suspension (titer: 1.26 x 10¹² vg/mL) was administered at a rate of 1 µL/min. After suturing the incision, analgesia was provided by administering 5 mg/kg of meloxicam (Boehringer Ingelheim, Georgia, USA). A total of 40 mice were used in each group and condition.

### Example 1.4. RNA extraction and quantitative reverse transcription-polymerase chain reaction

Brain tissues were homogenized on ice using TRIzol^{™} Reagent (ThermoFisher Scientific, Massachusetts, USA) to extract RNA. The extracted RNA pellets were cleaned, dried, and resuspended before applying DNase Enzyme (ThermoFisher Scientific, Massachusetts, USA) to remove any DNA traces. Quantification was carried out using a NanoDrop ND-1000 spectrophotometer (ThermoFisher, Massachusetts, USA). For RT-PCR, 1 µg of RNA per reaction was used with the High-Capacity cDNA Reverse Transcription Kit with RNase inhibitor (ThermoFisher Scientific, Massachusetts, USA). RT-PCR settings were as follows: 25°C for 10 min, 37°C for 120 min, and 85°C for 5 min. The resulting *hEPM2B* cDNA was used as a template for quantitative RT-PCR (RT-qPCR), performed using TaqMan^{™} Fast Advanced Master Mix (ThermoFisher Scientific, MA, USA). Probes used included *Epm2b* (ThermoFisher Scientific, MA, USA; Mm00614667_s1; Cat: #4331182), *EPM2B* (ThermoFisher Scientific, MA, USA; Hs01112790_s1; Cat: #4331182) and *Gapdh* (ThermoFisher Scientific, MA, USA; Mm99999915_g1; Cat: #4448491). qPCR conditions were: 50°C for 2 min, 95°C for 2 min, followed by 40 cycles of 95°C for 1 sec and 60°C for 20 sec. Triplicates of each sample were analyzed, and results were calculated using the 2^{-ΔΔCT} method.

### Example 1.5. PAS-diastase staining and immunohistochemistry

PAS-diastase (PAS-D) and immunohistochemistry (IHC) procedures were performed. Sections were rehydrated with decreasing concentrations of alcohol. For PAS-D staining, they were treated with porcine pancreas α-amylase (5 mg/ml in dH2O) (Merck, Darmstadt, Germany), processed with the PAS Kit (Merck, Darmstadt, Germany), and counterstained with Gill No. 3 hematoxylin (Merck, Darmstadt, Germany). For IHC, sections were incubated in 0.1M citrate buffer, pH 6 at 95°C for epitope unmasking, treated with H₂O₂ for 20 minutes to block endogenous peroxidase, and incubated with primary antibodies against green fluorescent protein (GFP) (1:100 dilution; Abcam, Cambridge, UK; Cat. #ab183734), Neuronal nuclei (NeuN) (1:100 dilution; Millipore, Temecula, CA, USA; Cat. # MAB377), ionized calcium-binding adapter molecule 1 (Iba1) (1:100 dilution; ThermoFisher Scientific, MA, US; #MA536257), and glial fibrillary acidic protein (GFAP) (1:1000 dilution; Millipore, Temecula, CA, USA; Cat. #MAB360). After incubation, sections were treated with biotinylated secondary antibodies, stained with the Vectastain ABC kit (Vector Laboratories, Burlingame, CA, USA), visualized with diaminobenzidine (Dako Cytomation, CA, USA) and H₂O₂, and counterstaining with Carazzi hematoxylin (Panreac Quimica, Barcelona, Spain). Two consecutive sections per animal from 4-6 mice were analyzed in the CA1 and CA2-CA3 regions of the hippocampus and layers IV-V of the sensorimotor cortex (SMC). Immunoreactivity was quantified by two researchers using ImageJ software (NIH, Bethesda, MD, USA), with results reported as the means of both quantifications.

### Example 1.6. Western blot analyses

Samples from the mouse brain (hippocampus and cortex) were lysed in RIPA buffer [50 mM Tris-HCl, pH 8; 150 mM NaCl; 0.5% sodium deoxycholate; 0.1% SDS; 1% Nonidet P40; 1mM PMSF; and a complete protease inhibitor cocktail (Roche, Barcelona, Spain)] for 30 min at 4°C with occasional vortexing. The lysates were passed ten times through a 25-gauge needle in a 1 ml syringe and centrifuged at 13,000 x g for 15 min at 4°C. Supernatants were collected and 35 µg protein was subjected to SDS-PAGE and transferred onto a PVDF membrane. Membranes were blocked in 5% (w/v) nonfat milk in Tris-buffered saline (TBS-T: 50 mM Tris-HCl, 150 mM NaCl, pH 7.4; with 0.1% Tween-20) for 1 h at room temperature and then incubated overnight at 4°C with the corresponding primary antibodies: anti-NF-kB (Santa Cruz Biotechnologies, Sc-8008), anti-caspase 8 (Cell Signaling, 9746), anti-phospho(Ser345)-MLKL (Millipore MABC1158), anti-CXCL10 (Abcam, ab9938) and anti-Lipocalin2 (Abcam, ab63929). Anti-Actin (Sigma, A2066) was used as a housekeeping antibody. Membranes were then probed with suitable secondary antibodies for 1 h at room temperature. Signals were detected using chemiluminescence with ECL Prime Western Blotting Detection Reagents (Cytiva-Amersham, RPN2232), and imaged using the Fuji-LAS-4000 system (GE Healthcare, Barcelona, Spain). The results were analyzed using the software Image Studio Lite version 5.2 (LI-COR Biosciences, Germany). Experiments were performed on at least three individuals from each genotype, including both males and females.

### Example 1.7. Object recognition task

The object recognition task (ORT) was used to analyze episodic memory. It involved habituating mice into a dark open field box for 10 minutes. After a 2-hour break, two identical objects, A and B, were placed in the center of the box. The mice were allowed to explore both objects freely, and the time spent examining each object (tA and tB) was recorded. The same test was repeated 2 hours later, but object B was replaced with a new object, C. The mice's exploration times (tA and tC) were measured using virtual timers generated by XNote Stopwatch software, which were activated when the mice inspected the object from a distance of 2 centimeters or less. The discrimination index (D.I.) was calculated using the formula: D.I. = (tC - tA) / (tC + tA).

### Example 1.8. Motor coordination

To evaluate the balance and motor coordination of mice, the rotarod test (Harvard Apparatus, Holliston, MA, USA) was used. After two days of training, only those mice that could stay on the cylinder for at least 60 seconds were included in the analysis. The latency time to fall from the rotating cylinder was measured twice a day, with each session lasting a maximum of five minutes, as the speed increased from 4 rpm to 40 rpm.

### Example 1.9. Spontaneous locomotor activity

Spontaneous movements of mice were monitored using a computerized actimeter (Harvard Apparatus, Holliston, MA, USA). The device detects breaks in an infrared light beam and records mouse crossings in an open field. SEDACOM 1.4 software (Harvard Apparatus, Holliston, MA, USA) was used to analyze spontaneous, rearing, and stereotyped movements at 5, 10, 15, 30, 45, and 60 minutes.

### Example 1.10. Video-EEG recording

A plastic pedestal with trimmed electrodes (Plastics1, Roanoke, VA, USA) was surgically implanted onto the skull and secured with acrylic resin. To alleviate post-surgical pain, meloxicam (5 mg/kg) (Boehringer Ingelheim, GA, USA) was administered to the animals. Following the surgery, mice were given one week to recover before video-EEG testing began. The recordings were performed using a wireless transmitter (Epoch, BIOPAC Systems, Inc., Goleta, CA, USA) attached to the pedestal, and data were digitally recorded on a computer under free-motion conditions. Mice were observed in their cages for 2 days under basal conditions. Afterwards, they were recorded for 30 minutes following PTZ injections. The recordings were done at a sampling rate of 250 Hz, and a 50 Hz notch filter was applied. Digital video cameras were used to capture their behavior. The EEG data were analyzed both automatically and manually using Acknowledge□ 5.0 software (Epoch, BIOPAC Systems, Inc., Goleta, CA, USA). Any signal loss or artifacts were identified and excluded. Power spectra were calculated using a spectral estimator derived from autoregressive processes, ensuring normalized amplitudes for consistent peak-to-peak ranges. The Comb Band Stop filter and a Blackman window were then applied. Automated seizure analysis was conducted, which included monitoring interictal epileptiform discharges (IEDs) and seizures.

### Example 1.11. Sensitivity to PTZ

PTZ (Merck, Darmstadt, Germany) was administered intraperitoneally to mice at subconvulsive doses (30 mg/kg). This treatment was used to assess the percentage of mice exhibiting myoclonic jerks. The study lasted for 45 min, and each animal was examined by two different researchers.

### Example 1.12. Statistical analysis

For behavioral tests, PTZ-based studies, and electrophysiology, a minimum of 12 mice per genotype were examined. For video-EEG and RT-qPCR assays, 4-6 mice per genotype were used. For immunohistochemistry and Western blot experiments, 4-8 mice per genotype were analyzed. The values are presented as the mean ± standard error of the mean (SEM) or as percentages. To assess differences between experimental groups, one- or two-way ANOVA, Fisher's exact test, the non-parametric Kruskal-Wallis test, or the Mann-Whitney test were used, as appropriate for each specific case. For EEG analysis, the Area Under the Curve (AUC) was calculated to compare differences in the power spectra between groups. All statistical analyses were conducted using GraphPad Prism 8.0 (San Diego, CA, USA). All statistical tests were two-tailed, and the statistical significance thresholds were set at *p < 0.05, **p < 0.01, ***p < 0.001, and ****p < 0.0001.

### Example 1.13. Intravenous injections

At 1 month of age, WT and *Epm2b*^{*-*/*-*} mice were anesthetized with 4% isoflurane and received a single IV injection into the retro-orbital venous sinus with rAAVP31-h*EPM2B* or rAAVP31-Null vectors. The total volume injected was 50 µL, with a viral titer of 5 x 10¹¹ vg/ml. Results were analyzed 5 months post-injection. For the study, 3 experimental groups were established: a group of WT mice injected with rAAVP31-Null, a group of *Epm2b*^{*-*/*-*} mice injected with rAA VP31-h*EPM2B*, and a group of *Epm2b*^{*-*/*-*} injected with rAAVP31-Null. Between 25 and 35 mice in each group and condition were used.

### Example 1.14. Electrophysiology

### Brain slicing

Mice were sacrificed by cervical dislocation. The brain was collected and immersed in ice-cold artificial cerebrospinal fluid (ACSF) containing (in mM): 126 NaCl, 2.5 KCl, 1.2 MgCl2, 1.2 NaH2PO4, 2.4 CaCl2, 10 glucose, and 25 NaHCO3, continuously bubbled with 95% O2 and 5% CO2, pH 7.4. Hippocampal transverse slices (400 µm thick for extracellular recordings) and cortico-striatal coronal slices (220-µm thick for patch-clamp recording) were obtained using a vibratome (LEICA, VT 1200S). Hippocampal brain slices were maintained in iced ACSF solution during the cutting process, while striatal brain slices were maintained in ACSF solution, bubbled with a 95% O2-5% CO2 gas mixture, at room temperature. Once cut, the slices were transferred to a recovery chamber with oxygenated ACSF at 30 °C for 30 minutes and then kept at room temperature (RT) for 1-2 more hours before experimental recordings. Each slice was then transferred to the recording chamber and submerged in ACSF at a constant rate of 2.5-3 mL/min at 29°C for hippocampus and at RT for the striatum.

### Extracellular recording

For extracellular recordings, the stimulating electrode was inserted into the perforant path fibers, and the recording electrode, made of borosilicate glass capillaries filled with 2 M NaCl (resistance 10-15 MΩ), was placed in the dentate gyrus (DG) close to the granular layer. Stimuli of 0.1Hz, 10 ms duration, and 20-30V amplitude evoked field excitatory postsynaptic potentials (fEPSPs), which in the DG included a population spike (PS) that reached 50% of maximum amplitude. The PS amplitude was defined as the average of the amplitude from the early positive peak to the negative peak, and from the negative peak to the late positive peak. An Axoclamp 2B amplifier (Molecular Devices, USA) was used for extracellular recordings. Traces were filtered at 3 KHz, digitized at 10KHz, and stored on a PC.

To induce LTP in the DG hippocampal region, a high-frequency stimulation (HFS) protocol was delivered, consisting of three trains of 1 s each with 5 min intervals, at a frequency of 100 Hz, after acquiring a stable baseline for 10 min.

### Evaluation of epileptic-like activity in DG

Epileptic-like activity in hippocampal DG was induced by perfusing slices with a Mg2+-free external solution to remove the magnesium block from NMDA glutamate receptors, in presence of bicuculline to antagonize GABA_{A} receptors. The epileptic-like activity was measured as the mean number of the PS and as PS amplitude (%). Specifically, for each experiment, the individual sweeps were averaged, yielding an average trace every two minutes. For each average trace obtained, the sum of the amplitudes of the PSs (considering from the second peak onwards) was normalized to the amplitude of the first peak, and the value was reported as a percentage. Thus, a time course of the amplitude of the PSs in percentage was obtained.

### Whole-cell patch-clamp recording

Whole-cell patch-clamp recordings were performed from medium spiny neurons (MSNs) visualized using infrared differential interference contrast microscopy (Olympus) in the dorsolateral striatum. Whole-cell patch-clamp recordings (access resistance: 15-30 MΩ; holding potential: -80 mV) were performed using a Multiclamp 700B amplifier (Molecular Devices) with borosilicate glass pipettes (4- 7 MΩ) filled with an internal solution containing 145 mM K+-gluconate, 0.1 mM CaCl2, 2 mM MgCl2, 0.1 mM EGTA, 10 mM HEPES, 0.3 mM Na-GTP and 2 mM Mg-ATP, adjusted to pH 7.3 with KOH. Access resistance was monitored online throughout each experiment, and recordings were discarded if either access resistance or holding current increased by more than 25% during the experiment. No liquid junction potential correction was applied. Membrane capacitance and resistance were measured online using the membrane seal test function of pClamp 10.7 (-5 mV step, 10 msec).

MSNs were identified by their hyperpolarized resting membrane potential (RMP, ~ -80 mV), absence of spontaneous action potential discharge at rest, and presence of tonic firing activity during current-induced membrane depolarization. When recording spontaneous excitatory postsynaptic currents (sEPSCs), picrotoxin (50 µM) was added to the artificial cerebrospinal fluid (ACSF) to block GABA_{A} currents. Data were acquired with pClamp 10.7 (Molecular Devices), with currents filtered at 0.1 kHz and digitized at 200 µs using Clampex 10.7. The data were analyzed offline using the automatic detection and subsequently checked manually for accuracy. For MSNs synaptic plasticity recordings, a bipolar electrode, connected to a stimulation unit was placed in the white matter between the cortex and the striatum to stimulate glutamatergic fibres (0.1 Hz) and evoked excitatory postsynaptic currents (eEPSCs), while the recording electrode was placed in the dorsolateral striatum. After recording eEPSCs of stable amplitudes for at least 10 min, a high-frequency stimulation (HFS) protocol, consisting of three trains of 3 s (20 s intervals), was delivered at 100 Hz to induce LTP. External Mg2+ ions were omitted from the solution to maximize the contribution of NMDA receptors during LTP experiments. In all patch-clamp experiments, 50 µM picrotoxin was added to the Krebs solution to block GABA_{A} receptors.

### Example 1.15. Biochemical parameters

Glucose levels, liver biochemical parameters and immunoglobulin profile, including aspartate transaminase (AST), alanine transaminase (ALT), alkaline phosphatase (AP), immunoglobulin G (IgG) and immunoglobulin M (IgM), were assessed using a Roche Cobas autoanalyzer.

### EXAMPLE 2. RESULTS

### Example 2.1. ICV injections of the rAAV-hEPM2B or rAAV-GFP vectors enable efficient brain transgene transcription and translation

Three and nine months after a single ICV injection of rAAV-h*EPM2B* or rAAV-Null vectors into the brains of 3-month-old *Epm2b*^{*-*/*-*} and WT mice, the expression of the *hEPM2B* transgene was measured using RT-qPCR **(****Figure 1A**, **B****).** The results showed that levels of *hEPM2B* transcripts in *Epm2b*^{*-*/*-*} mice, at both 3 and 9 months post-injection, were comparable to the levels of *Epm2b* transcripts in WT animals. Due to the lack of effective antibodies for detecting the malin protein, we used a rAAV2/9 vector with the CAG promoter carrying the *GFP* reporter gene to analyze vector biodistribution in the central nervous system. Three months after ICV injection of the rAAV-*GFP* vector in WT mice, immunohistochemical analysis revealed widespread GFP expression throughout the hippocampus, cortex and fimbria (Figure 1C). This indicates that a single ICV injection of the rAAV-h*EPM2B* or rAAV-GFP vectors enables the transcription of the *hEPM2B* or *GFP* transgenes and the translation of the GFP protein (and, by inference, the malin protein) within the central nervous system of *Epm2b*^{*-*/*-*} and WT mice.

### Example 2.2. rAAVP31-hEPM2B transduction following IV administration in Epm2b^{-/-}mice leads to expression of the hEPM2B transgene within CNS cells and other organs

Transcription of the *hEPM2B* transgene was quantified via RT-qPCR, 5 months after a single IV injection of the *rAAVP31-hEPM2B* vectors in *Epm2b*^{*-*/*-*} mice **(****Figure 2****).** The presence of *hEPM2B* **(****Figure 2****)** transcript was confirmed throughout the CNS, including cerebellum, striatum, cortex and hippocampus, brainstem and spinal cord, as well as in other organs such as the liver and heart, but not in the quadriceps femoris. The expression levels of *hEPM2B* transcripts were above the endogenous expression levels of the *Epm2b* gene in WT mice **(****Figure 2****)**

### Example 2.3. IV injections of rAAVP31-hEPM2B vectors diminish astrogliosis, slightly reduce neurodegeneration and decrease LB formation in Epm2b^{-/-} mice

The effect of early systemic administration of the rAAVP31-h*EPM2B* vectors on neuroinflammation in *Epm2b*^{*-*/*-*} **(****Figure 3A****)** mice was assessed 5 months after IV injections using a GFAP antibody. Quantification of GFAP-positive cells in the CA1 field of the hippocampus revealed a reduction in GFAP-immunostained cells in treated *Epm2b*^{*-*/*-*} **(****Figure 3B****)** mice compared to those treated with the Null vector, indicating a decrease in astrogliosis. Astrogliosis was further examined in the CA2-CA3 regions of the hippocampus **(****Figure 4A**, **B****).** A reduction in astrogliosis was observed in the CA2-CA3 regions of *Epm2b*^{*-*/*-*} mice treated with the rAAVP31-h*EPM2B* vector compared to those receiving the Null vector **(****Figure 4A**, **B****).** These findings suggest that rAAVP31-h*EPM2B* vector may effectively reduce neuroinflammation in specific regions, with varied responses across different brain areas.

The effect of the rAAVP31-h*EPM2B* vectors on neurodegeneration was examined using NeuN immunostaining 5 months post-IV injection **(****Figure 3A****, C;** **Figure 4A, C****).** A slight trend toward a decrease in NeuN-positive cells was observed in the CA1 and CA2-CA3 hippocampal regions of 6-month-old *Epm2b*^{*-*/*-*} mice treated with the rAAVP31-Null vector, though these differences were not statistically significant compared to WT mice. Notably, this trend was absent in *Epm2b*^{*-*/*-*} mice treated with the rAAVP31-h*EPM2B* vectors, where NeuN-positive cell counts were restored to levels comparable to WT.

We analyzed the number of LBs in the CA1 and CA2-CA3 regions of the hippocampus, as well as in layers IV-V of the sensorimotor cortex, using PAS-D staining in 6-month-old mice treated with rAAVP31-h*EPM2B* and rAAVP31-Null vectors. A significant reduction in the number of LB was observed in the CA1 **(****Figure 3A, D****)** and CA2-CA3 regions **(****Figure 4A****,** **D****)** of the hippocampus in *Epm2b*^{*-*/*-*}mice following treatment with rAAVP31-h*EPM2B,* compared to those receiving the rAAVP31-Null vector.

### Example 2.4. Treatment with rAAV-hEPM2B ICV administered reduces astrogliosis and microgliosis

To assess the effects of rAAV-h*EPM2B* administration on neuroinflammation in *Epm2b*^{*-*/-}mice, we quantified GFAP- and Iba1-positive cells in the hippocampus **(****Figure 5****;** **Figure 6****).** *Epm2b*^{*-*/*-*} mice treated with rAAV-Null displayed increased astrogliosis (measured as the presence of the GFAP marker) in various hippocampal regions 3 and 9 months post-ICV injection, with significant differences observed 9 months after injection **(****Figure 5****).** Treatment with the rAAV-h*EPM2B* vector showed a tendency to reduced astrogliosis in the CA1 region of the hippocampus, reaching similar levels to those seen in WT mice 9 months post-treatment **(****Figure 5A, C****).**

We also analyzed microgliosis in *Epm2b⁻*/*⁻* mice 3 and 9 months post-injection **(****Figure 6****).** The hippocampus of *Epm2b*^{*-*/*-*} mice treated with the rAAV-hEPM2B vector displayed a reduction in microgliosis compared to those injected with the rAAV-Null vector 9 months after injection. This reduction was observed in both the CA1 **(****Figure 6A-C****)** and CA2-CA3 **(****Figure 6D-F****)** regions of the hippocampus.

### Example 2.5. Treatment with rAAV-hEPM2B decreases the levels of neuroinflammation markers present in Epm2b^{-/-} mice

Neuroinflammation has recently been identified as a significant and novel feature of LD, contributing to its pathogenesis and progression. To obtain a deeper understanding of the potential beneficial effects of rAAV-h*EPM2B* on various neuroinflammatory mediators in LD, we analyzed the levels of caspase 8, the phosphorylated form of the mixed lineage kinase domain-like (pMLKL) protein, NF-kB, chemokine CXCL10, and lipocalin 2 (Lcn2), as described in (43, 44), in cortical and hippocampus extracts from WT mice injected with rAAV-Null and *Epm2b*^{*-*/*-*} mice treated with either rAAV-h*EPM2B* or rAAV-Null. Three and nine months post-injections, *Epm2b*^{*-*/*-*} mice injected with rAAV-Null exhibited increased levels of these markers in the cortex and hippocampus compared to WT mice **(****Figure 7****;** **Figure 8****).** Interestingly, *Epm2b*^{*-*/*-*} mice treated with rAAV-h*EPM2B* showed a reduction in these neuroinflammatory markers to levels similar to those found in WT mice. This indicates that administration of rAAV-h*EPM2B* effectively reduced neuroinflammation as early as 3 months post-ICV administration **(****Figure 7****),** and this effect was sustained up to 9 months after treatment **(****Figure 8****).**

### Example 2.6. rAAV-hEPM2B vector-based gene therapy reverses episodic memory alterations and improves spontaneous locomotor activity, but does not correct motor coordination impairments

ORT was performed to evaluate the episodic memory state in *Epm2b*^{*-*/*-*}mice 3 and 9 months after the administration of rAAV-h*EPM2B* or rAAV-Null vectors **(****Figure 9A, B****).** Memory performance was significantly improved in *Epm2b*^{*-*/*-*}mice 3 months after treatment to levels comparable to WT mice, as indicated by the recovery of the D.I. between the old and new object **(****Figure 9A****).** However, 9 months after treatment **(****Figure 9B****),** the differences between WT and *Epm2b*^{*-*/*-*} mice injected with the rAAV-Null or rAAV-h*EPM2B* vectors were not statistically significant. Despite this, *Epm2b*^{*-*/*-*}mice treated with the rAAV-h*EPM2B* vector showed a tendency to recover the mild memory deficits present in *Epm2b*^{*-*/*-*} mice **(****Figure 9B****).**

Assessment of motor coordination with the rotarod test revealed that *Epm2b*^{*-*/*-*}mice treated with rAAV-h*EPM2B* or rAAV-Null vectors remained on the rod for a shorter period than WT mice 3 months after injection **(****Figure 9C****),** although no differences were found 9 months after treatment administration **(****Figure 9D****).** Examination of spontaneous motor activity showed that rAAV-h*EPM2B* treatment led to an improvement in spontaneous accumulated, stereotyped and rearing movements 3 months post-injection **(****Figure 9E****).** *Epm2b*^{*-*/*-*} treated mice presented higher spontaneous locomotor activity than WT control mice, indicating some level of hyperactivity **(****Figure 9E****).** However, 9 months after treatment, *Epm2b*^{*-*/*-*} mice treated with rAAV-h*EPM2B* showed no differences in locomotor activity in comparison to rAAV-Null treated mice **(****Figure 9F****).**

### Example 2.7. Electrophysiological studies in the DG and striatum of WT and Epm2b^{-/-}mice following IV injections of rAAVP31-hEPM2B vectors

We performed an electrophysiological investigation in two brain regions that are significantly involved in seizure events as well as in the mechanisms underlying procedural and associative memory: the nucleus striatum and the hippocampus.

We first investigated the effect of gene therapy on the basal membrane properties of striatal medium spiny neurons (MSN). MSNs from *Epm2b*^{*-*/*-*} mice displayed significantly lower membrane resistance compared to their WT counterpart, while membrane capacitance showed an increasing trend **(****Figure 10E****).** Furthermore, the analysis of the current-voltage relationship revealed a significant increase in inward currents elicited at hyperpolarizing voltage steps, while no differences in resting membrane potential were observed between *Epm2b*^{*-*/*-*} and WT mice **(****Figure 10F****).** The number of action potentials (APs) was significantly reduced in *Epm2b*^{*-*/*-*} mice compared to WT mice **(****Figure 10G****).** This reduction was particularly evident during the initial steps of depolarizing current injection, which triggered significantly fewer action potentials in the *Epm2b*^{*-*/*-*} animals **(****Figure 10G****).** We did not observe any significant alterations in either rheobase current or threshold potential compared to WT **(****Figure 10H****).** As shown in the histograms, gene therapy treatment with rAAVP31-*hEPM2B* did not alter the physiological membrane properties of MSNs. Interestingly, treatment of *Epm2b*^{*-*/*-*} mice with the *rAAVP31-hEPM2B* vector completely restored the basal membrane properties of MSNs to physiological levels **(****Figure 10E-H****).**

To further investigate cortico-striatal transmission, we examined excitatory postsynaptic currents (sEPSCs) and LTP of MSNs. We found a significant increase in sEPSC frequency, along with a noticeable, albeit not statistically significant, trend toward increase sEPSC amplitude in *Epm2b*^{*-*/*-*} mice **(****Figure 11C****).** Moreover, LTP of MSNs remained at physiological levels in *Epm2b*^{*-*/*-*} mice **(****Figure 11D****).** Gene therapy with the rAAVP31-*hEPM2B* vector fully normalized sEPSC frequency, although it did not appear to affect sEPSC amplitude.

Using extracellular recordings of field potentials, we mimicked epileptic-like activity in vitro, on hippocampal DG slices, by evoking repetitive population spikes (PS) in the presence of 0.1 µM bicuculline in an Mg²⁺-free solution as described previously. Under these experimental conditions, mild epileptic-like activity was induced in control mice. Our data showed increased epileptic-like activity in the DG slices of *Epm2b*^{*-*/*-*} mice compared to WT, as revealed by the elevated PS amplitude **(****Figure 12B****).** In contrast, the DG of *Epm2b*^{*-*/*-*} mice displayed a milder epileptic-like phenotype compared to other Lafora disease models mice. rAAVP31-h*EPM2B* reduced epileptic-like activity in *Epm2b*^{*-*/*-*} mice; however, the PS amplitude in *Epm2b*^{*-*/*-*} + rAAVP31-h*EPM2B* mice was not statistically different from either WT mice or *Epm2b*^{*-*/*-*} + rAAVP31-Null mice **(****Figure 12B****).** We also examined synaptic plasticity by inducing LTP in DG granule cells. LTP in *Epm2b*^{*-*/*-*} mouse DG was maintained at physiological levels **(****Figure 12D****).**

### Example 2.8. Effects of intravenous administration of the rAAVP31-hEPM2B vector on liver function and immune system

We examined potential liver-related adverse effects, specifically lobular inflammation and hepatic steatosis in 6-month-old WT and *Epm2b*^{*-*/*-*} mice, 5 months after IV injection of the rAAVP31-Null or rAAVP31-h*EPM2B* vectors, using H&E staining. No signs of lobular inflammation were observed in WT mice IV injected with the rAAVP31-Null vector or in *Epm2b*^{*-*/*-*} mice treated with the rAAVP31-h*EPM2B* vectors **(****Figure 13****).** However, a slight, though not statistically significant, increase in lobular inflammation was noted in *Epm2b*^{*-*/-}mice treated with the rAAVP31-Null vector compared to WT control mice **(****Figure 13****),** suggesting potential liver alterations in these models. Additionally, we found no evidence of steatosis or hepatocyte hypertrophy **(****Figure 13****).** Nonetheless, more in-depth studies are required to evaluate possible hepatic abnormalities in these models.

We also analyzed biochemical parameters in the serum of mice 15 months after vectors administration to evaluate potential long-term liver damage or immune responses **(****Figure 14****).** Our findings indicate that *Epm2b*^{*-*/*-*} mice exhibited significantly lower glucose levels compared to WT mice. Treatment with the *rAAVP31-hEPM2B* vector, however, resulted in a normalization of glucose levels **(****Figure 14****).** Liver function was assessed by measuring levels of the enzymes ALP, AST and ALT, which are commonly used indicators of hepatic health. The vector did not caused alterations in the level of the hepatic enzymes AST and ALT. Finally, to assess potential immune activation, we measured serum levels of IgG and IgM. No significant differences were observed between mice treated with the rAAVP31-h*EPM2B* vectors, WT mice, and untreated mice **(****Figure 14****).** These results suggest that the IV administration of the rAAVP31-h*EPM2B* vector does not induce a long-term adaptative immune response.

## Claims

1. A vector comprising a nucleic acid sequence coding for EPM2B protein.

2. Vector, according to claim 1, **characterized in that** it is a nucleic acid-based vector, nanoparticle-based vector, extracellular vesicles or cell-derived vector.

3. Vector, according to any of the previous claims, wherein the nucleic acid-vector is a non-viral nucleic acid vector or a viral nucleic acid vector.

4. Vector, according to any of the previous claims, wherein the nucleic acid-vector is a non-viral nucleic acid vector selected from: plasmids, BACs, YACs, mRNA or saRNA, or a viral nucleic acid vector selected from: adenovirus, lentivirus, adenovirus, retrovirus or HSV.

5. Vector, according to any of the previous claims, wherein EPM2B protein is encoded by a nucleic acid sequence comprising the sequence SEQ ID NO: 1.

6. Vector, according to any of the previous claims, wherein EPM2B protein comprises the amino acid sequence SEQ ID NO: 2.

7. Vector, according to any of the previous claims, **characterized by** comprising the SEQ ID NO: 3.

8. Vector, according to any of the previous claims, wherein the nucleic acid sequence encoding EPM2B is operably linked to a regulatory sequence that drives the expression of the coding sequence.

9. Vector, according to any of the previous claims, wherein the vector is a non-integrative vector.

10. Vector, according to any of the previous claims, wherein the vector is an adeno-associated virus-based non-integrative vector, preferably derived from a serotype 2 or 9 adeno-associated virus.

11. Vector, according to any of the previous claims, wherein the capsid of the adeno-associated virus-based vector is made of a capsid proteins of the serotype 9 adeno-associated virus, and the nucleic acid sequence contained in the capsid is flanked at both ends by internal terminal repeats corresponding to serotype 2 adeno-associated virus.

12. Vector, according to any of the previous claims, wherein the vector comprises a regulatory sequence which is a constitutive promoter, preferably CAG.

13. Vector, according to any of the previous claims, for use a as medicament.

14. Vector, according to any of the previous claims, for use in a method for the treatment of Lafora disease.

15. Vector, according to any of the previous claims, for use in a method for the treatment of Lafora disease wherein the method comprises the administration of the vector to a presymptomatic subject diagnosed with or predisposed to Lafora disease.
